# EUROPEAN PATENT SPECIFICATION

(11) **EP 1 797 123 B1**
(45) Date of publication and mention of the grant of the patent: **03.03.2010**
(21) Application number: 05797206.9
(22) Date of filing: 06.10.2005
(51) Int. Cl.: C07K 14/775, A61K 38/04, C07K 16/18, G01N 33/50

(54) **COMPLEMENTARY PEPTIDES FOR ß-AMYLOID 29-42 PEPTIDE**
KOMPLEMENTÄRE PEPTIDE FÜR ß-AMYLOID 29-42-PEPTID
PEPTIDES COMPLÉMENTAIRES POUR BETA-AMYLOID 29-42 PEPTIDE

(30) Priority: 08.10.2004 EP 04077735
(43) Date of publication of application: 20.06.2007
(73) Proprietor: Université de Liège, 4020 Liège (BE); Faculté Universitaire des Sciences Agronomiques de Gembloux, 5030 Gembloux (BE)
(72) Inventor: HEINEN, Ernst, B-4453 Villers St Siméon (BE); BRASSEUR, Robert, B-5351 Haillot (BE); DECAFFMEYER, Marc, B-1350 Marilles (BE)
(74) Representative: Polypatent
(86) International application number: PCT/EP2005/055050
(87) International publication number: WO 2006/037800

(56) References cited:
- WO-A-03/086326
- LINS L ET AL: "Molecular determinants of the interaction between the C-terminal domain of Alzheimer's beta-amyloid peptide and apolipoprotein E alpha-helices." JOURNAL OF NEUROCHEMISTRY. AUG 1999, vol. 73, no. 2, August 1999 (1999-08), pages 758-769, XP002306814 ISSN: 0022-3042 cited in the application
- WANG GUANGSHUN ET AL: "Conformations of human apolipoprotein E(263-286) and E(267-289) in aqueous solutions of sodium dodecyl sulfate by CD and 1H NMR" BIOCHEMISTRY, vol. 35, no. 32, 1996, pages 10358-10366, XP002306815 ISSN: 0006-2960

### SEQUENCE LISTING

<110> Faculté universitaire des Sciences Agronomiques de Gembloux Université de Liège
<120> Complementary peptides for β-Amyloid 29-42 peptide
<130> P21527WO
<150> EP 04 077 735.1
<151> 2004-10-08
<160> 8
<170> PatentIn version 3.1
<210> 1
   <211> 14
   <212> PRT
   <213> Homo sapiens
<400> 1
   Gly Ala Ile Ile Gly Leu Met Val Gly Gly Val Val Ile Ala
   1               5                   10
<210> 2
   <211> 18
   <212> PRT
   <213> Homo sapiens
<400> 2
   Glu Asp Met Gln Arg Gln Trp Ala Gly Leu Val Glu Lys Val Gln
   Ala
   1               5                   10                  15
   Ala Val
<210> 3
   <211> 18
   <212> PRT
   <213> Artificial
<220>
   <221> VARIANT
   <222> (1) .. (18)
   <223> artificial peptide
<400> 3
   Glu Asp Met Gln Arg Gln Leu Ala Gly Val Val Glu Lys Val Gln Ala
   1               5                   10                  15
   Ala Val
<210> 4
   <211> 18
   <212> PRT
   <213> Artificial
   <220>
   <221> PEPTIDE
   <222> (1)..(18)
   <223> artificial peptide
<400> 4
   Glu Asp Met Gln Arg Gln Leu Ala Gly Leu Val Glu Lys Trp Gln Ala
   1               5                   10                  15
   Ala Val
<210> 5
   <211> 18
   <212> PRT
   <213> Artificial
   <220>
   <221> PEPTIDE
   <222> (1) .. (18)
   <223> artificial peptide
<400> 5
   Glu Asp Met Gln Arg Gln Leu Ala Gly Met Trp Glu Lys Val Gln Ala
   1               5                   10                  15
   Ala Val
<210> 6
   <211> 18
   <212> PRT
   <213> Artificial
   <220>
   <221> PEPTIDE
   <222> (1) .. (18)
   <223> artificial peptide
<400> 6
   Glu Asp Val Gln Arg Gln Leu Ala Gly Leu Val Glu Lys Val Gln Ala
   1 5 10 15
   Ala Val
<210> 7
   <211> 18
   <212> PRT
   <213> Artificial
   <220>
   <221> PEPTIDE
   <222> (1)..(18)
   <223> artificial peptide
<400> 7
   Glu Asp Met Gln Arg Gln Met Ala Gly Leu Met Glu Lys Met Gln Ala
   1 5 10 15
   Ala Val
<210> 8
   <211> 18
   <212> PRT
   <213> Artificial
   <220>
   <221> PEPTIDE
   <222> (1) .. (18)
   <223> artificial peptide
<400> 8
   Glu Asp Met Gln Arg Gln Val Ala Gly Met Trp Glu Lys Val Gln Ala
   1 5 10 15
   Ala Val

## Description

The present invention refers to peptides derived from the wild type sequence of apolipoprotein E3 fragments complementary to the C-terminal fragment of the β-Amyloid 29-42 fusion peptide involved in Alzheimer's disease.

**Technical field**

Until now, Alzheimer's disease can only be detected when the first symptoms appear, while, in fact, the disease has developed itself over a long period of time (anywhere from 1 to 20 years).

This disease is caused by the transconformation of a normal protein (β-Amyloid or Aβ) into a toxic form. The role of the β-Amyloid 1-42 protein in the formation of senile plaques associated with Alzheimers disease has been described by various authors: Shoji, M. (2002) Front Biosci. 7, 997-1006; Galasko, D. (1998) J. Neural Transm. Suppl 53, 209-221; Gooch, M. D. and Stennett, D. J. (1996) Am. J. Health Syst. Pharm. 53, 1545-1557.

It is also known, that the β-Amyloid 29-42 or Aβ 29-42 peptide (as numbered from the initial methionine) is a fusion peptide, *i.e.,* an inductor of membrane fusion because of its tilted properties (Pillot, T., Goethals, M., Vanloo, B., Talussot, C., Brasseur, R., Vandekerckhove, J., Rosseneu, M., and Lins, L. (1996) J.Biol.Chem. 271, 28757-28765). Aβ 29-42 is a tilted peptide.

The term "tilted peptides" refers to peptides that are short (10-20 residues) and which present an assymetric hydrophobicity gradient along their sequence under their helical form. This special hydrophobic profile allows the peptides to be inserted in a phospholipid bilayer with typical insertion angles ranging from 30° to 60°. This tilted orientation is thought to destabilise membranes and induce processes such as fusion.

A tilted peptide can be detected using the procedure of molecular modelling described in Brasseur, R. Mol. Membr. Biol. 17: 31-40 (2000). Briefly, a peptide is considered as tilted if it shows the following properties: the peptide is 11 to 18-20 amino acids long; its mean hydrophobicity (as calculated by the Eisenberg consensus scale) is higher than 0.1 when the peptide is built as an a helix, the angle between the helix axis and the interface plane is between 30° and 60° and the hydrophobic isopotential envelopes are asymmetric. An important characteristic of a tilted peptide is its ability to induce liposome fusion in *in vitro* experiments.

**Background of the invention**

The Alzheimer β-Amyloid 29-42 peptide is a tilted peptide with the following amino-acid sequence: GAIIGLMVGGVVIA (SEQ ID NO: 1). It is known that this peptide is able to induce the fusion of lipid vesicles (Pillot, T., Goethals, M., Vanloo, B., Talussot, C., Brasseur, R., Vandekerckhove, J., Rosseneu, M., and Lins, L. (1996) J. Biol. Chem. 271, 28757-28765). This suggests that a direct interaction of the β-Amyloid peptide with cell membranes might account for part of its cytotoxicity.

Apolipoprotein E polymorphism influences the pathology of Alzheimer's disease. The existence of several types of interaction between the apoE isoforms and the Alzheimer β-Amyloid 29-42 peptide is known. Lins *et al*. hightlighted the specificity of the interaction between β-Amyloid 29-42 and Apo E3 as well as the types of interactions involved (Lins, L., Thomas-Soumarmon, A., Pillot, T., Vandekerchkhove, J., Rosseneu, M., and Brasseur, R. (1999) J. Neurochem. 73, 758-769). The best interaction (in terms of energy and interaction surface) observed by Lins *et al.* occurs between the Alzheimer β-Amyloid 29-42 peptide and the apolipoprotein E3 wild type peptide having the amino-acid sequence EDMQRQWAGLVEKVQAAV (SEQ ID NO: 2), spanning from residue 270 to residue 287, as numbered from the initial methionine (ApoE 270-287). The hydrophobic contribution to the interaction between Aβ29-42 and ApoE270-287 is crucial as compared to the electrostatic and Van der Waals contributions. For the early diagnosis of Alzheimer's disease and the development of treatments, it is highly desirable to design and use peptides having a further improved interaction with the target peptide, namely Aβ29-42.

**Objects of the invention**

An object of the present invention was to provide complementary peptides derived from the apolipoprotein E3 (270-287) wild type peptide having an improved interaction stability with the β-Amyloid 29-42 peptide.

Another object of the invention is to provide such peptides which, when forming a complex with the β-Amyloid 29-42 peptide, have an improved antifusogenic effect on the Aβ 29-42 peptide. This antifusogenic activity prevents the tilted peptide Aβ 29-42 from inducing membrane fusion.

**Definitions**

As used herein, the following terms must be understood according to the definitions given below.

The term "Aβ 29-42" refers to the C-terminal part of the β-Amyloid protein of sequence GAIIGLMVGGVVIA (SEQ ID NO: 1). The term "ApoE WT" refers to the wild type Apolipoprotein E3 peptide 270-287 of sequence EDMQRQWAGLVEKVQAAV (SEQ ID NO: 2). The term "ApoE mutant n" refers to the peptide n derived from ApoE WT. The term "[Aβ 29-42/ ApoE WT] complex" refers to the complex of Aβ 29-42 and ApoE WT. The term "[Aβ 29-42/ ApoE mutant n] complex" refers to the complex of Aβ 29-42 and the peptide n derived from ApoE WT. The term "complementary peptide sequences" refers to peptide sequences selected from the group consisting of SEQ ID NOs 3, 4, 5, 6, 7 and 8 or any peptide derived therefrom, because they bind to the β-Amyloid peptidic fragment 29-42 (Aβ 29-42). The term "complementary peptide" refers to peptides having SEQ ID NOs 3, 4, 5, 6, 7 and 8 or any peptide derived therefrom. The terms variant, chemical derivative, peptidomimetic, (direct) label are defined hereunder.

**Summary of the invention**

The object of the present invention was solved by an isolated peptide derived from the wild type Apolipoprotein E3 peptidic fragment 270-287 (ApoE WT) of sequence EDMQRQWAGLVEKVQAAV (SEQ ID NO: 2), wherein the peptide is from 18 to 50 amino acid residues in length and having improved interaction with the β-Amyloid peptidic fragment 29-42 (Aβ 29-42) GAIIGLMVGGVVIA (SEQ ID NO: 1), said peptide comprising one or a combination of amino acid sequences selected from the group consisting of:
EDMQRQLAGVVEKVQAAV (SEQ ID NO: 3)
EDMQRQLAGLVEKWQAAV (SEQ ID NO: 4)
EDMQRQLAGMWEKVQAAV (SEQ ID NO: 5)
EDVQRQLAGLVEKVQAAV (SEQ ID NO: 6)
EDMQRQMAGLMEKMQAAV (SEQ ID NO: 7)
EDMQRQVAGMWEKVQAAV (SEQ ID NO: 8)
or a multimer thereof, or a variant thereof,
wherein 1 or 2 amino acid residues is/are deleted from the N- and/or C-terminus in SEQ ID NOs: 3, 4, 5, 6, 7 and 8, Like the peptides comprising the sequences SEQ ID NOs 3, 4, 5, 6, 7 or 8 said variants,
or multimers thereof also have improved properties compared to the apolipoprotein E3 (270-287) wild type peptide. The present invention provides peptides and recombinant proteins comprising those peptides, wherein said peptides are complementary peptides derived from the apolipoprotein E3 (270-287) wild type peptide having an improved interaction stability with the β-Amyloid 29-42 peptide. Further, these peptides preferably have an improved antifusogenic effect on the Aβ 29-42 peptide when forming a complex with the β-Amyloid 29-42 peptide. This antifusogenic activity prevents the tilted peptide Aβ 29-42 from inducing membrane fusion. These characteristics, features, properties and activities of the peptides also apply to the following embodiments of the present invention.

The peptide sequences having SEQ ID NOs 3, 4, 5, 6, 7 and 8, or said variant thereof will hereinafter also be called complementary peptides or complementary peptide sequences, because they bind to the β-Amyloid peptidic fragment 29-42 (Aβ 29-42).

The peptides of the invention may be used in a method for the diagnosis of Alzheimer's disease, in a kit for the detection of Aβ 29-42 peptides implicated in Alzheimer's disease, and for the manufacture of a medicament for preventing, treating or curing Alzheimer's disease.

In a preferred embodiment the peptide is from 18 to 40, more preferred from 18 to 30, even more preferred from 18 to 25, and most preferred 18 amino acid residues in length.

The isolated peptide of the present invention when complexed to the β-Amyloid peptidic fragment 29-42 (Aβ 29-42) GAIIGLMVGGVVIA (SEQ ID NO: 1), decreases the fusogenic activity of Aβ 29-42:

In a preferred embodiment the peptide comprises an amino acid sequence selected from the group consisting of: EDMQRQLAGWEKVQAAV (SEQ ID NO: 3)and EDMQRQMAGLMEKMQAAV (SEQ ID NO: 7)

The present invention also provides a recombinant protein, polypeptide or oligopeptide comprising one or a combination of amino acid sequences selected from the group consisting of:
EDMQRQLAGWEKVQAAV (SEQ ID NO: 3)
EDMQRQLAGLVEKWQAAV (SEQ ID NO: 4)
EDMQRQLAGMWEKVQAAV (SEQ ID NO: 5)
EDVQRQLAGLVEKVQAAV (SEQ ID NO: 6).
EDMQRQMAGLMEKMQAAV (SEQ ID NO: 7)
EDMQRQVAGMWEKVQAAV (SEQ ID NO: 8).
   In a preferred embodiment the oligopeptide is from 18 to 40, more preferred from 18 to 30, even more preferred from 18 to 25, and most preferred 18 amino acid residues in length.

The recombinant protein comprises any one of the sequences SEQ ID NOs 3, 4, 5, 6, 7 and 8, or said variant multimer thereof. In a preferred embodiment the recombinant comprises more than one of the sequences SEQ ID NOs 3, 4, 5, 6, 7 and 8, or said variant or multimer thereof. In case more than one of said complementary peptide sequences is present in the recombinant protein, the sequences either may be different from each other, or the complementary peptide sequence may be repeated. For example, a recombinant protein may comprise the complementary peptide sequence SEQ ID NO: 3 and the complementary peptide sequence SEQ ID NO: 4, or it may comprise the sequence SEQ ID NO: 3 twice. The moiety of the recombinant protein which does not cover said complementary peptide sequences serves as a carrier protein for said complementary peptide sequences. The recombinant protein preferably contains further sequences having a specific function; those sequences are preferably selected from the group comprising: epitopes for antibody binding, binding sequences for protein-protein interaction, binding sequences for ligand binding, and protein sequences having enzymatic activity. These further sequences having a specific function may serve as markers which can be used for detection and identification of the recombinant protein containing the complementary peptide sequence and by this for the detection of the β-Amyloid peptidic fragment 29-42 (Aβ 29-42) bound to it.

The present invention further provides a polynucleotide encoding the recombinant protein containing the complementary peptide sequence or a combination thereof.

The peptides of the present invention or the recombinant proteins comprising the complementary peptide sequences as lined out above can be used for the detection of β-Amyloid protein or its peptidic fragment β-Amyloid 29-42 (Aβ 29-42) GAIIGLMVGGVVIA (SEQ ID NO: 1).

Further, the present invention also provides a detection method of the β-Amyloid protein or its peptidic fragment β-Amyloid 29-42 (Aβ 29-42) GAIIGLMVGGVVIA (SEQ ID NO: 1), wherein the method comprises:
- a step of contacting a biological sample containing the β-Amyloid protein or its peptidic fragment β-Amyloid 29-42 (Aβ 29-42) with a peptide or recombinant protein comprising one or a combination of amino acid sequences selected from the group consisting of SEQ ID NOs 3, 4, 5, 6, 7 and 8 or a multimer thereof; or a variant thereof, wherein 1 or 2 amino acid residues is/are deleted from the N-and/or C-terminus in SEQ ID NOs: 3, 4, 5, 6, 7 and 8;
- a step of detecting the binding of said peptide or of said recombinant protein to the β-Amyloid protein or to its β-Amyloid peptidic fragment 29-42 (Aβ 29-42).

Further, the present invention also provides a kit for use in the detection of the β-Amyloid protein or its peptidic fragment β-Amyloid 29-42 (Aβ 29-42) GAIIGLMVGGVVIA (SEQ ID NO: 1), the kit comprises a peptide or recombinant protein comprising one or a combination of amino acid sequences selected from the group consisting of SEQ ID NOs 3, 4, 5, 6, 7 and 8 or a multimer thereof ; or a variant thereof, wherein 1 or 2 amino acid residues is/are deleted from the N-and/or C-terminus in SEQ ID NOs: 3, 4, 5, 6, 7 and 8; the kit further comprises an antibody for detecting the binding of said peptide or of said recombinant protein to the β-Amyloid protein or to its peptidic fragment β-Amyloid 29-42 (Aβ 29-42).

Further, the present invention also provides a binding assay for the detection of the β-Amyloid protein or its peptidic fragment β-Amyloid 29-42 (Aβ 29-42) GAIIGLMVGGVVIA (SEQ ID NO: 1), the assay involving the use of a peptide or recombinant protein comprising one or a combination of amino acid sequences selected from the group consisting of SEQ ID NOs 3, 4, 5, 6, 7 and 8 or a multimer thereof or a variant thereof, wherein 1 or 2 amino acid residues is/are deleted from the N-and/or C-terminus in SEQ ID NOs: 3, 4, 5, 6, 7 and 8. In a preferred embodiment the assay is an immuno-assay, a radioimmuno-assay, an enzyme-linked immunosorbent assay or a sandwich assay. In a further preferred embodiment the peptide (*i.e*., the complementary peptide) or the recombinant protein comprising the complementary peptide is bound to a solid carrier and the agent for detecting the binding of said peptide or of said recombinant protein to the β-Amyloid protein/peptidic fragment is an antibody which binds to the β-Amyloid protein or to its peptidic fragment β-Amyloid 29-42 (Aβ 29-42). In an alternative preferred embodiment the peptide (*i.e*., the complementary peptide) or the recombinant protein comprising the complementary peptide is solubilized and labelled with a direct label and the agent for detecting the binding of said peptide or of said recombinant protein to the β-Amyloid protein/peptidic fragment is an antibody bound to a solid carrier (for example a chromatographic strip), wherein the antibody binds to the β-Amyloid protein or to its peptidic fragment β-Amyloid 29-42 (Aβ 29-42).

As a typical example for a detection method, a sandwich assay can be mentioned. Here, a peptide or recombinant protein comprising a complementary peptide sequence according to the present invention is bound to a solid support (*e.g*., a protein binding surface, colloidal metal particles, iron oxide particles, latex particles and polymeric beads as described in U.S. Patent N° 6,689,566). A sample containing the analyte β-Amyloid protein or its peptidic fragment β-Amyloid 29-42 (Aβ 29-42)) is brought into contact with said support. The analyte will bind to the complementary peptide sequence of the present invention. Then the binding of the analyte can be determined by different means. For instance, the binding of said peptide or of said recombinant protein to the β-Amyloid protein/peptidic fragment can be determined by an antibody which binds to the β-Amyloid protein or to its peptidic fragment β-Amyloid 29-42 (Aβ 29-42). Alternatively, the peptide (*i.e*., the complementary peptide) or the recombinant protein comprising the complementary peptide is solubilized and labelled with a direct label. A sample containing the analyte (β-Amyloid protein or its peptidic fragment β-Amyloid 29-42 (Aβ 29-42)) is mixted with the solution and the labelled peptide or recombinant protein binds to the analyte. Upon application on a chromatographic strip, the aqueous mixture migrates towards a section where an antibody directed against the β-Amyloid protein/peptidic fragment is bound. After binding of the antibody to the β-Amyloid protein/peptidic fragment, the presence of the latter is visible by the directly labelled complementary peptide or the respective recombinant protein comprising a complementary peptide sequence complexed to the β-Amyloid protein/peptidic fragment.

The person skilled in the art will understand that there are many different possibilities for how to use the complementary peptides or recombinant proteins comprising the same for the detection of the β-Amyloid protein or its peptidic fragment β-Amyloid 29-42 (Aβ 29-42).

Further, the present invention also provides a medicament comprising one or more of the peptides or recombinant proteins or polynucleotides of the present invention. In a preferred embodiment the medicament comprises one or more peptides or recombinant proteins, comprising one or a combination of sequences selected from the group consisting of SEQ ID NOs 3, 4, 5, 6, 7 and 8, or a multimer thereof or a variant thereof, wherein 1 or 2 amino acid residues is/are deleted from the N-and/or C-terminus in SEQ ID NOs: 3, 4, 5, 6, 7 and 8. These peptides and recombinant proteins, respectively, show an improved interaction stability with the β-Amyloid 29-42 peptide. Further, these peptides preferably have an improved antifusogenic effect on the Aβ 29-42 peptide when forming a complex with the β-Amyloid 29-42 peptide. This antifusogenic activity prevents the tilted peptide Aβ 29-42 from inducing membrane fusion.

Further, the present invention also provides the use of the peptides or recombinant proteins or polynucleotides of the present invention for the manufacture of a medicament for preventive and/or therapeutical treatment of Alzheimer's disease.

The present invention also provides antibodies or antibody fragments binding to an antigen specific for an epitope sequence selected from the group consisting of SEQ ID NOs 3, 4, 5, 6, 7 and 8 or by a multimer thereof or a variant thereof, wherein 1 or 2 amino acid residues is/are deleted from the N-and/or C-terminus in SEQ ID NOs: 3, 4, 5, 6, 7 and 8; and wherein at least four amino acids in said peptide sequence are part of a reactive portion with said antibody. Such antibodies may be polyclonal, monoclonal, bispecific, chimeric or antiidiotypic, and preferably include antigen-binding fragments thereof. Any immunoassay known in the art may be used to detect the binding of such an antibody to a peptide, variant, chemical derivative or peptidomimetic thereof according to this invention. Antibodies of this invention are used to detect the presence of or measure the amount of the peptide epitope in a biological material or other sample by direct or competitive immunoassay. The antibodies can be coupled to a solid support and used in affinity chromatography to isolate and purify material containing the peptide epitope. Conversely, the peptide, variant, chemical derivative, or peptidomimetic thereof of this invention, bound to a solid support, is used to enrich or purify specific antibodies. Antiidiotypic antibodies can be used to gain knowledge of the structure of a peptide, variant or chemical derivative of this invention when bound to a receptor for it.

The present invention further provides the use of an antibody or antibody fragment specific for a peptide sequence selected from the group consisting of SEQ ID NOs 3, 4, 5,2, 7, and 8, or a multimer thereof, or a variant thereof wherein 1 or 2 amino acid residues is/are deleted from the N-and/or C-terminus in SEQ ID NOs: 3, 4, 5, 6, 7 and 8; and wherein at least four amino acids in said peptide sequence are part of a reactive portion with said antibody; wherein said antibody is used in a binding assay for the detection of the β-Amyloid protein or its peptidic fragment β-Amyloid 29-42 (Aβ 29-42) GAIIGLMVGGWIA (SEQ ID NO: 1).

In a preferred embodiment the invention provides the use of an antibody or antibody fragment specific for a peptide sequence selected from the group consisting of SEQ ID NOs 3, 4, 5, 6, 7, and 8, or a multimer thereof, or a variant thereof, wherein 1 or 2 amino acid residues is/are deleted from the N-and/or C-terminus in SEQ ID NOs: 3, 4, 5, 6, 7 and 8; and wherein at least four amino acids in said peptide sequence are part of a reactive portion with said antibody; wherein said antibody is used in a detection method of the β-Amyloid protein or its peptidic fragment β-Amyloid 29-42 (Aβ 29-42) GAIIGLMVGGWIA (SEQ ID NO: 1); wherein said method comprises:
- a step of contacting a biological sample containing the β-Amyloid protein or its peptidic fragment β-Amyloid 29-42 (Aβ 29-42) with a peptide or recombinant protein comprising one or a combination of amino acid sequences selected from the group consisting of SEQ ID NOs 3, 4, 5, 6, 7, and 8, or a multimer thereof; or a variant thereof, wherein 1 or 2 amino acid residues is/are deleted from the N-and/or C-terminus in SEQ ID NOs: 3, 4, 5, 6, 7 and 8; and
- a step of detecting the binding of said peptide or of said recombinant protein to the β-Amyloid protein or to its β-Amyloid peptidic fragment 29-42 (Aβ 29-42).

The present invention also provides the above mentioned product, use or method,
wherein two or more residues are added to the C-terminus after Val in SEQ ID NO: 3, 4, 5, 6, 7 and 8, wherein these residues are selected from the group consisting of Leu-(Gly)ₙ, Ile-(Gly)ₙ, Val-(Gly)ₙ, Nva-(Gly)ₙ, or Nle-(Gly)ₙ, wherein Nva is norvaline, Nle is norleucine, and n=1-10, and/or wherein one or more residues is/are added to the N-terminus before Glu in SEQ ID NO: 3, 4, 5, 6, 7 and 8, wherein these residues are selected from the group consisting of Gly, Lys-(Gly)ₙ, Tyr-(Gly)_{n,} or Gly-(Gly)ₙ, wherein n=1-10.
Preferably, any one of the following amino acids in form of a 9-mer addition is added to the C-terminus after Val in SEQ ID NO: 3, 4, 5, 6, 7 and 8 Leu, Ile, Val, Nva, Nle, Met, Ala, and Gly. The present invention also provides the above mentioned product, use or method,
wherein the peptides according to SEQ ID NO: 3, 4, 5, 6, 7 and 8 are derivatized by the following derivatizations selected from the group:
i) Lysinyl and amino terminal residues are derivatized with succinic or other carboxylic acid anhydrides, and/or
ii) α-amino-containing residues are derivatized with a substance selected from the group consisting of imidoesters such as methyl picolinimidate; pyridoxal phosphate; pyridoxal; chloroborohydride; trinitrobenzenesulfonic acid; O-methylisourea; 2,4 pentanedione; and transaminase-catalyzed reaction with glyoxylate;
iii) selectively modifying the carboxyl side aspartyl or glutamyl residues with a substance selected from the group consisting of carbodiimides (R-N=C=N-R') such as 1-cyclohexyl-3-(2-morpholinyl-(4-ethyl) carbodiimide or 1-ethyl-3-(4-azonia-4,4-dimethylpentyl) carbodiimide;
iv) aspartyl and glutamyl residues are converted to asparaginyl or glutaminyl residues by reaction with ammonia;
v) hydroxylation of proline and lysine residues;
vi) phosphorylation of hydroxyl groups of seryl or threonyl residues,
vii) methylation of the amino group of lysine,
viii) acetylation of the N-terminal amine,
ix) amidation of the C-terminal carboxyl groups.
The present invention also provides the above mentioned product, use or method, wherein the peptides according to SEQ ID NO: 3, 4, 5, 6, 7 and 8 are replaced by its respective retro-inverso sequence, wherein the direction of the peptide chain has been inverted and wherein all the amino acids belong to the D-series.

The invention will be illustrated by the following examples in light of the appended figures, wherein:

Figure 1 shows Accessible Surface Area (ASA) as a function of ApoE WT residues in the [Aβ 29-42/ ApoE WT] complex and alone (in Å²).

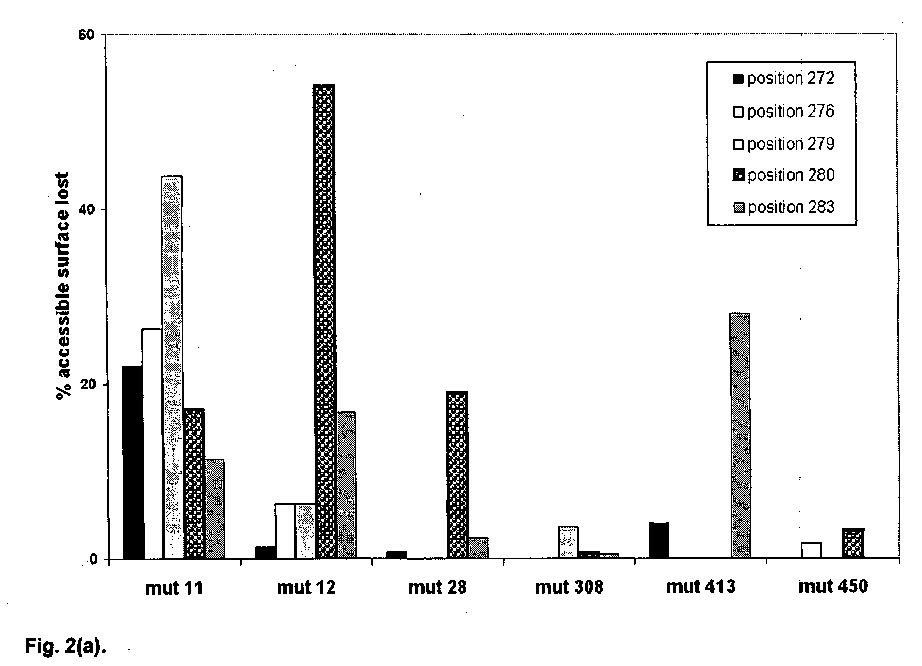

Figure 2 (a) shows ASA lost for each mutated residue of the selected [Aβ 29-42/ ApoE WT mutant n] complexes as compared to the same mutant alone.

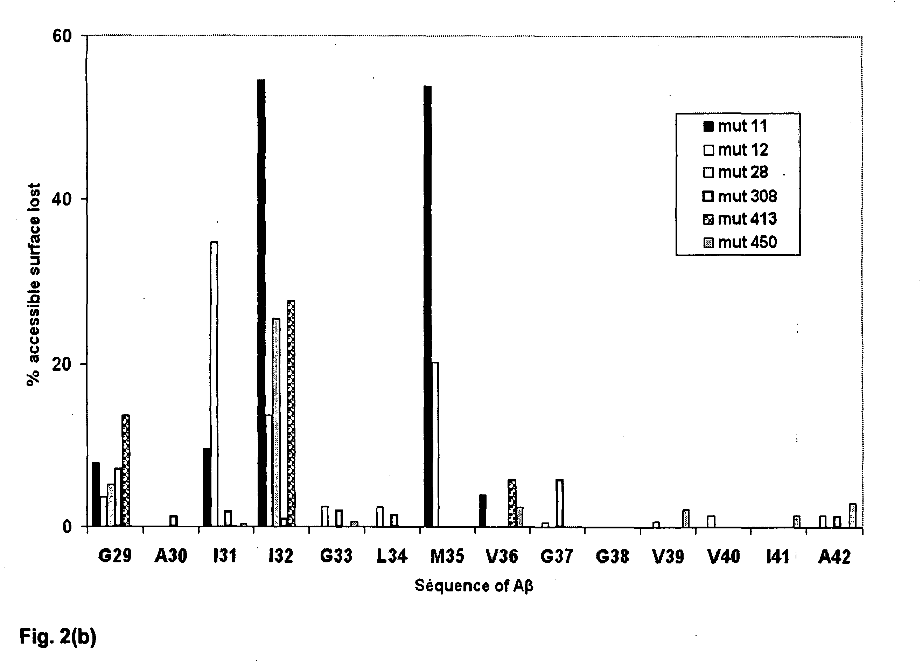

Figure 2 (b) shows ASA lost for each residue of the Aβ 29-42 peptide in interaction with the considered ApoE mutant n as compared to Aβ 29-42 alone.

Figure 3 shows lipid fusion induced by Aβ 29-42 and inhibitory effect after addition of ApoE WT, ApoE mutant 11 and ApoE mutant 413 monitored by fluorescence at room temperature.

Figure 4 (a) shows leakage assays of liposomal content induced by Aβ 29-42 and inhibitory effect after addition of ApoE mutant 11. Liposomes are the same as for fusion assays. □ (open rectangle) = Aβ 29-42 alone; ■ (closed rectangle) = ApoE mutant 11 /Aβ 29-42 (R=1).

Figure 4 (b) shows leakage assays of liposomal content induced by Aβ 29-42 and inhibitory effect after addition of mutant 413. Liposomes are the same as for fusion assays. □ (open rectangle) = Aβ 29-42 alone; ■ (closed rectangle) = ApoE mutant 413/Aβ 29-42 (R=1).

Figure 5 shows the lost accessible surface area (ASA) as a function of ApoE 270-287 residues in the complex with Aβ 29-42 and alone (in %).

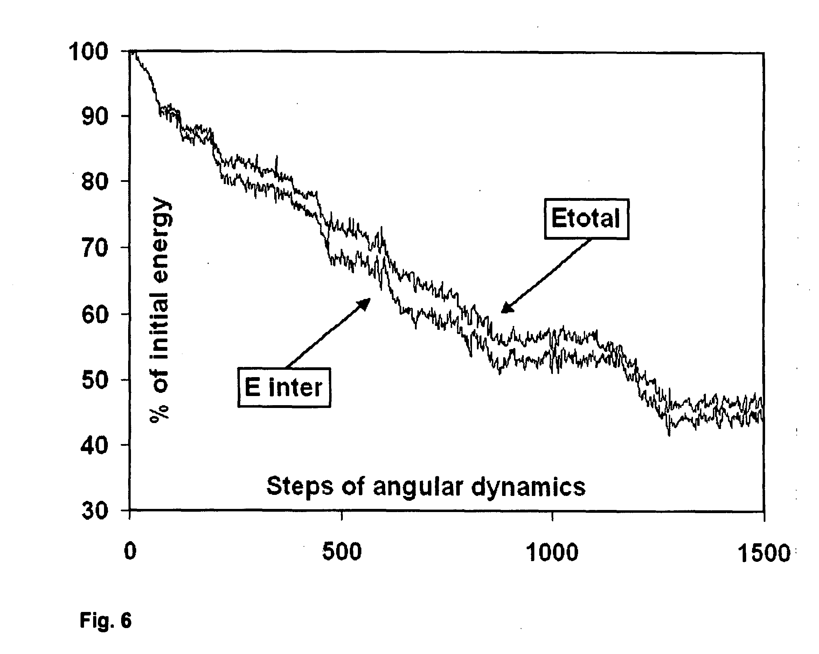

Figure 6 shows the evolution of the energy of inter and total (inter plus intra) molecule interactions of the peptide of SEQ ID NO:3 and Aβ29-42 complex during the procedure of Monte Carlo and angular dynamics. The procedure lasts for few minutes and does not show significant energy decrease if allowed to proceed.

Figure 7 shows the cell viability (%) of SH-SY5Y cells in presence of increasing concentrations of Aβ 1-42, Aβ 1-40 or Aβ 25-35 peptides.

Figure 8 shows the cell viability (%) of SH-SY5Y cells in presence of increasing concentrations of ApoE 270-287 peptide.

Figure 9 shows the cell viability (%) of SH-SY5Y cells in presence of the ApoE 270-287 (2µM) and Aβ 1-42 (100µM) peptides.

**Production of the Peptides of the Invention**

The peptides of the current invention can, for example, be synthesized or produced using recombinant methods and techniques known in the art. Although specific techniques for their preparation are described herein, it is to be understood that all appropriate techniques suitable for production of these peptides are intended to be within the scope of this invention.

Generally, these techniques include DNA and protein sequencing, cloning, expression and other recombinant engineering techniques permitting the construction of prokaryotic and eukaryotic vectors encoding and expressing each of the peptides of the invention.

In one mode, the peptides may be prepared by peptide synthesis according to method described in Biotechnology and Applied Biochem., 12:436 (1990) or by methods described in Current Protocols in Molecular Biology, Eds. Ausubel, F.M., et al., John Wiley & Sons, N.Y. (1987). Further, the peptides of the present invention may be synthesized and purified by a number of established procedures known in the art such as the so-called "Merrifleld" solid phase peptide synthesis described in Merrifield, J. Am. Chem. Soc. 85:2149-2154 (1963). Solid phase synthesis techniques have been provided for the synthesis of several peptide sequences on substrates such as "pins" (*See*, Geysen et al., J. Immun. Meth. 102:259-274 (1987)). Other solid-phase techniques involve synthesis of various peptide sequences on different cellulose disks supported on a column (*See*, Frank and Doring, Tetrahedron 44:6031-6040). Peptides may also be synthesized using automated peptide synthesizers, *e.g.* Peptide Synthesizer-Model 431-A (Applied Biosystems).

The peptides of the invention may be produced by expression of a nucleic acid encoding a peptide of interest, or by cleavage from a longer length polypeptide encoded by the nucleic acid. Expression of the encoded polypeptides may be done in bacterial, yeast, plant, insect, or mammalian hosts by techniques well known in the art.

In an embodiment, a peptide of interest of the invention is obtained by cloning the DNA sequence into a vector starting with a DNA codon for methionine inserted upstream of 5' to the first DNA codon of the desired peptide sequence and modifying the DNA codon corresponding to the last amino acid of a desired peptide to a stop codon by mutagenesis techniques known in the art. A host cell is transformed with the modified nucleic acid to allow expression of the encoded peptide. In a further embodiment, the cloned DNA is engineered to create a proteolytic cleavage site within the polypeptide. The polypeptide is then cleaved after production in the host to generate the peptide of interest. Examples of mutagenesis techniques include, for example, methods described in Promega Protocols and Applications GWde, Promega Corp, Madison, WI, p. 98 (1991) or according to *Current Protocols in Molecular Biology,* supra.

If the peptide is to be synthesized *via* a prokaryotic vector, the DNA sequence encoding the peptide of the invention preferably does not contain a signal peptide sequence. In addition, a DNA codon for methionine (Met) is typically inserted upstream of 5' to the first DNA codon of the coding sequence.

The peptides of the invention may be produced as an hybrid or a fusion protein made with a peptide of the present invention, resulting in the recombinant protein comprising the complementary peptide sequence. As for example, a peptide of the present invention may be produced as fused with the maltose binding protein. For this, the DNA fragment encoding the peptide is cloned into the pMAL-C2x plasmid so that an in-frame fusion protein between the maltose binding protein and the peptide is produced.

Methods for cloning DNA into a vector and for inserting, deleting and modifying polynucleotides and for site directed mutagenesis are described, for example, in *Promega Protocols and Applications Guide, supra.* Cells or bacteria may be transfected with a vector, preferably with an expression vector having the desired DNA sequence attached thereto, by known techniques including heat shock, electroporation, calcium phosphate precipitation and lipofection, among others. The terminal peptides or other analogues or fragments may then be extracted and purified by, for example, high pressure liquid chromatography (HPLC), ion exchange chromatography or gel permeation chromatography. However, other methods and techniques known in the art of conducting the different steps or combinations of these steps necessary to derive the peptide of this invention or equivalent steps are contemplated to be within the scope of this invention.

The phrase "substantially purified" or "isolated" when referring to a peptide or protein, means a chemical composition which is essentially free of other cellular components. It is preferably in a homogeneous state although it can be in either a dry or aqueous solution. Purity and homogeneity are typically determined using analytical chemistry techniques such as polyacrylamide gel electrophoresis or high performance liquid chromatography. A protein which is the predominant species present in a preparation is substantially purified. Generally, a substantially purified or isolated protein will represent more than 80% of all macromolecular species present in the preparation. Preferably, the protein is purified to represent greater than 90% of all macromolecular species present More preferably the protein is purified to greater than 95%, and most preferably the protein is purified to essential homogeneity, wherein other macromolecular species are not detected by conventional techniques.

Also included in this invention are additional variants wherein two or more residues are added to the C-terminus after Val in SEQ ID NO: 3, 4, 5, 6, 7 and 8 or said variant thereof. These residues may be Leu-(Gly)ₙ, Ile-(Gly)ₙ, Val-(Gly)ₙ, Nva-(Gly)ₙ, or Nle-(Gly)ₙ, wherein Nva is norvaline, Nle is norleucine, and n=1-10.

Also included in this invention are addition variants wherein one or more residues is/are added to the N-terminus before Glu in SEQ ID NO: 3, 4, 5, 6, 7 and 8 or said variant thereof. These residues may be Gly, Lys-(Gly)ₙ, Tyr-(Gly)ₙ, or Gly-(Gly)ₙ, wherein n=1-10. Another preferred derivative of this invention is a 9-mer addition variant wherein any one of the following amino acids is added to the C-terminus after Val in SEQ ID NO: 3, 4, 5, 6, 7 and 8, or said variant thereof: Leu, Ile, Val, Nva, Nle, Met, Ala, and Gly.

In general, preferred peptide addition variants may have up to about 30 additional amino acids, more preferably about 20, most preferably 11.

The functional limitations placed on the peptide variant, and the ease by which these activities can be tested using conventional means, would permit one skilled in the art to ascertain whether such modification would affect the peptide's activity. In view of the structural description provided herein, it would be easy to one of ordinary skills in the art to determine whether a peptide variant falls within the scope of this invention.

**Chemical Derivatives**

"Chemical derivatives" of SEQ ID NO: 3, 4, 5, 6, 7 or 8 contain additional chemical moieties not normally a part of the peptide. Covalent modifications of the peptide are included within the scope of this invention. Such modifications may be introduced into the molecule by reacting targeted amino acid residues of the peptide with an organic derivatizing agent that is capable of reacting with selected side chains or terminal residues.

Lysinyl and amino terminal residues are derivatized with succinic or other carboxylic acid anhydrides. Derivatization with a cyclic carboxylic anhydride has the effect of reversing the charge of the lysinyl residues. Other suitable reagents for derivatizing α-amino-containing residues include imidoesters such as methyl picolinimidate; pyridoxal phosphate; pyridoxal; chloroborohydride; trinitrobenzenesulfonic acid; O-methylisourea; 2,4 pentanedione; and transaminase-catalyzed reaction with glyoxylate.

Carboxyl side groups, aspartyl or glutamyl, may be selectively modified by reaction with carbodiimides (R-N=C=N-R') such as 1-cyclohexyl-3-(2-morpholinyl-(4-ethyl) carbodiimide or 1-ethyl-3-(4-azonia-4,4-dimethylpentyl) carbodiimide. Furthermore, aspartyl and glutamyl residues can be converted to asparaginyl and glutaminyl residues by reaction with ammonia.

Other modifications include hydroxylation of proline and lysine, phosphorylation of hydroxyl groups of seryl or threonyl residues, methylation of the amino group of lysine (Creighton, *supra,* pp. 79-86), acetylation of the N-terminal amine (Ac-), and amidation of the C-terminal carboxyl groups (-Am).

For every single peptide sequence disclosed herein, this invention includes the corresponding retro-inverso sequence wherein the direction of the peptide chain has been inverted and wherein all the amino acids belong to the D-series. For example, the retro-inverso analogue of the natural L-series peptide EDMQRQLAGVVEKVQAAV (SEQ ID NO: 3) is VAAQVKEVVGALQRQMDE which is composed of D-series amino acids and in which E is the N-terminus and V is the C-terminus. For example the retro-inverso analogue of the natural L-series capped peptide Ac-EDMQRQLAGWEKVQAAV-Am is Ac-VAAQVKEWGALQRQMDE-Am which is composed of D-series amino acids and in which the N-terminal E is acetylated and the C-terminal V is amidated. The complete range of N-terminal capping groups and the complete range C-terminal capping groups specified for the L-series peptides are also intended for the D-series peptides.

Also included are peptides wherein one or more D-amino acids has/have been substituted for one or more L-amino acids. Additionally, modified amino acids or chemical derivatives of amino acids may be provided such that the peptide contains additional chemical moieties or modified amino acids not normally a part of a natural protein. Such derivatized moieties may improve the solubility, absorption, biological half life, and the like. Moieties capable of mediating such effects are disclosed, for example, in Remington's Pharmaceutical Sciences, 16th ed., Mack Publishing Co., Easton, Pa. (1980).

**Multimeric Peptides**

The present invention also includes longer peptides in which the basic peptidic sequence of about 16-20 amino acids is repeated from about two to about 100 times, with or without intervening spacers or linkers. For example, a multimer of the peptide EDMQRQLAGWEKVQAAV (SEQ ID NO: 3) is shown by the following formula (EDMQRQLAGWEKVQAAV-Xm)ₙ-EDMQRQLAGVVEKVQAAV wherein m=0 or 1, n=1-100. X is a spacer group, preferably C1-C20 alkyl, C1-C20 alkenyl, C1-C20 alkynyl, C1-C20 polyether containing up to 9 oxygen atoms or Glyz(z=1-10).

It is understood that such multimers may be built from any of the peptide variants, chemical derivative or peptidomimetic described herein. Moreover, a peptide multimer may comprise different combinations of peptide monomers, *i.e.,* SEQ ID NO: 3, 4, 5, 6, 7 or 8 and the disclosed variants, chemical derivative or peptidomimetic thereof. Such oligomeric or multimeric peptides can be made by chemical synthesis or by recombinant DNA techniques as discussed herein. When produced chemically, the oligomers preferably have from 2-8 repeats of the basic peptide sequence. When produced recombinantly, the multimers may have as many repeats as the expression system permits, for example from two to about 100 repeats.

All the foregoing peptides, variants and chemical derivatives including multimeric peptides must have the binding activity of the peptides of the invention, *e.g.,* bind to the Aβ 29-42 peptide. Alternatively, or in addition, the peptide, variant or chemical derivatives should compete with labelled peptides of the invention for binding to a ligand or binding partner for the peptides of the invention, *e.g*., the Aβ 29-42 peptide.

Additional Discussion of Peptides and Variants

Minor modifications of the amino acid sequence might affect activity if those modifications are selected purely at random. However, one skilled in the art of peptide and peptidomimetic design would follow a well-established set of "rules" in creating useful variants and derivatives. According to Bowie et al. (Science 247:1306-1310 (1990)), if a particular property of a side chain, such as charge or size, is important at a given position, only side chains that have the required property will be allowed. Conversely, if the chemical identity of the side chain is unimportant, then many different substitutions will be permitted. Studies based on these notions revealed that proteins are surprisingly tolerant of amino acid substitutions (Bowie *et al., supra* at page 1306). Thus, the art recognizes and accepts certain types of changes in proteins and in peptides. Such acceptable modifications delineate a genus of peptides wherein each species predictably has the requisite type and/or level of activity.

Further, Bordo and Argos (J. Mol. Biol. 217:721-729 (1991)) reported a statistical analysis of protein sequences and provided guidelines for "safe" amino acid substitutions in protein design, and by analogy, peptide design. It is axiomatic that proteins with similar functions are topographically similar at least in those regions responsible for activity.

In addition to applying this topographical criterion to the design and production of peptides with sequence homology or acceptable sequence substitutions, this criterion can be used as a basis for generating chemical derivatives of the peptides of the invention. This is fundamental to structure-based drug design and modeling. Although the solution structure of a free peptide may not exactly mimic its bound conformation, the solution structure does provide a starting scaffold for optimizing derivatives which mimic the peptide's activity. In fact, such scaffolds could not be derived in the absence of the basic topographical information about this peptide, either free or bound. If a derivative is prepared with a structure/topography similar to that of the peptides of the invention and the requisite biological and binding activity as disclosed herein, then it is within the scope of the present invention.

If a peptide is designed in accordance with this invention based on either the sequence or the topography (structure) of any one of the peptides of the invention, and it has the bioactivity stated above, then it must be similar in conformation to the peptides of the invention and therefore falls within the scope of the invention. The assessment of activity in bioassays or binding assays such as those described herein is routine in the art and is the logical way to determine whether a compound is active. A useful substitution variant, addition variant or other chemical derivative of the peptides of the invention is a compound that has been designed based on the sequence or topographical structure of any one of the peptides of the invention.

Systematic approaches in the art that allow the optimization of a peptide and development of peptidomimetics (Hruby et al., Biochem J. 268:249-262 (1990); and Hruby, 1993, supra) flow from a single starting point: the identification of a peptide lead compound. The peptide and peptidomimetic design approaches disclosed herein and/or known in the art for generating an optimized compound are not possible without first identifying an active lead peptide, so that these designed peptides or peptidomimetics constitute a genus of compounds "around" the original peptide. Schemes for preparing active derivatives of the parent peptide have been described (*e.g*., Moore et al., Adv. Pharmacol. 33:91-141 (1995); Giannis and Rubsam, Adv. Drug Research 29:1-78 (1997)). Although each approach may require some experimentation, it is neither random nor undue. By..following accepted schemes practiced by those skilled in the art, one can generate families of similarly acting compounds.

**Antibodies directed to the peptides of the invention**

Antibodies raised against peptides of the invention can be used to detect the presence of those peptides in various assays. Preferred assays are enzyme immunoassays or radioimmunoassay. The following references (incorporated by reference in their entirety) describe the production, purification, testing and use of antibodies: Hartlow, E. et al., Antibodies: A Laboratory Manual, Cold Spring Harbor Laboratory Press, Cold Spring Harbor, N.Y., 1988; Campbell, A., In: Laboratory Techniques in Biochemistry and Molecular Biology, Volume 13 (Burdon, R., et al., eds.), Elsevier, Amsterdam (1984)); Work, T. S. et al., Laboratory Techniques and Biochemistry in Molecular Biology, North Holland Publishing Company, NY, 1978; Weintraub, B., Principles of Radioimmunoassays, Seventh Training Course on Radioligand Assay Techniques, The Endocrine Society, March, 1986; Butler, J. E. (ed.), Immunochemistry of Solid-Phase Immunoassay, CRC Press, Boca Raton, 1991; Butler, J. E., In: STRUCTURE OF ANTIGENS, Vol. 1, Van Regenmortel, M., ed., CRC Press, Boca Raton 1992, pp. 209-259; Butler, J. E., In: van Oss, C. J. et al., (eds), IMMUNOCHEMISTRY, Marcel Dekker, Inc., New York, 1994, pp. 759-803; Voller, A. et al. (eds)., Immunoassays for the 1980's, University Park Press, Baltimore, 1981.

**Labels used in detection assays**

As examples of labels that can be used in detection assays, the peptide can be labelled for detection using fluorescence-emitting metals such as ¹⁵² Eu, or others of the lanthanide series. These metals can be attached to the peptide using such metal chelating groups as diethylenetriaminepentaacetic acid (DTPA) or ethylenediaminetetraacetic acid (EDTA). The peptide can be made detectable by coupling it to a chemiluminescent compound. The presence of the chemiluminescent-tagged peptide is then determined by detecting the presence of luminescence that arises during the course of a chemical reaction. Examples of particularly useful chemiluminescers are luminol, isoluminol, theromatic acridinium ester, imidazole, acridinium salt and oxalate ester. Likewise, a bioluminescent compound may be used to label the peptide. Bioluminescence is a type of chemiluminescence found in biological systems in which a catalytic protein increases the efficiency of the chemiluminescent reaction. The presence of a bioluminescent protein is determined by detecting the presence of luminescence. Important bioluminescent compounds for purposes of labelling are luciferin, luciferase and aequorin.

It is also possible to use chromogenic compounds to perform colorimetric detection of the peptides of the invention. This mode of detection is based on chromogenic compounds (chromophores) with high extinction coefficients. It is also well understood that a combination of complementary peptides, or proteins carrying one or more complementary peptides, can be used in those assays.

**Nucleic Acids of the Invention**

Also provided herein are isolated nucleic acids that comprise DNA or RNA sequences (polynucleotides) encoding the peptides of the invention. The nucleic acids of the invention may further comprise vectors for expression of the peptides of the invention. It is understood by one of ordinary skill in the art that because of degeneracy in the genetic code, substitutions in the nucleotide sequence may be made which do not result in changes in the encoded amino acid sequence. It is further understood by one of ordinary skill in the art that both complementary strands of any DNA molecule described herein are included within the scope of the invention.

**Treatment Protocols**

The treatment of Alzheimer's disease comprises administering to a patient an effective amount of one or more peptides of the invention, or recombinant proteins carrying the respective complementary peptide sequence. The following description refers to the use of peptides. However, it is understood that the explanations also apply to recombinant proteins carrying the complementary peptides. As used herein, the term "treatment" is intended to refer to the prevention, amelioration, or reduction in severity of a symptom or combination of symptoms of Alzheimer's disease. Similarly, an effective dose of a peptide of the invention is a dose sufficient to prevent, ameliorate, or reduce the severity of a symptom of Alzheimer's disease.

The peptides of the invention may be administered singly or in combination with each other or other agents used for the therapy of Alzheimer's disease. Typically, the peptides of the invention are administered in an amount of about 8 micrograms to 3,000 µg/kg per day, and more preferably about 20 to 1,500 µg/kg per day preferably once or twice daily. However, other amounts, including substantially lower or higher amounts, may also be administered. The peptides of the invention are administered to a human subject in need of treatment intramuscularly, subcutaneously, intravenously, intratumorally, by any other acceptable route of administration.

Different amounts of the peptide may also be administered as seen suitable by a practitioner for specific cases. For this or any other application the peptide of this invention may be administered in an amount of about 10 to 3,750 µg/kg, and more preferably about 15 to 1,600 µg/kg. Any means of administration is suitable. The foregoing ranges are, however, suggestive, as the number of variables in regard to an individual treatment regime is large, and considerable excursions from these recommended values are expected.

The peptide(s) contained in the composition can be protected against degradation by proteases *in vivo* by a number of methods known to one skilled in the art, *e.g.,* by increasing the concentration of peptides in the peptide composition; more frequent administration of the peptide composition; and chemical protection of peptide(s) using protective groups well known to those skilled in the art of peptide chemistry. Such protective groups will preferably protect the peptide from the effects of proteases and will not interfere with the peptide-binding reaction.

The peptide of the present invention may be used in combination with other compositions and procedures for treatment of diseases.

**Gene Therapy**

Gene therapy utilizing recombinant DNA technology to deliver nucleic acids encoding peptides of the invention into patient cells or vectors which will supply the patient with gene product *in vivo* is also contemplated within the scope of the present invention.

Gene therapy techniques have the potential for limiting the exposure of a subject to a gene product, such as polypeptide, by targeting the expression of the therapeutic gene to a tissue of interest. For example, WIPO Patent Application Publication No. WO 93/15609 discloses the delivery of interferon genes to vascular tissue by administration of such genes to areas of vessel wall injury using a catheter system. In another example, an adenoviral vector encoding a protein capable of enzymatically converting a prodrug, a "suicide gene", and a gene encoding a cytokine are administered directly into a solid tumor.

Other methods of targeting therapeutic genes to tissues of interest include the three general categories of transductional targeting, positional targeting, and transcriptional targeting (for a review, *see, e.g*., Miller et al. FASEB J. 9:190-199 (1995)). Transductional targeting refers to the selective entry into specific cells, achieved primarily by selection of a receptor ligand. Positional targeting within the genome refers to integration into desirable loci, such as active regions of chromatin, or through homologous recombination with an endogenous nucleotide sequence such as a target gene. Transcriptional targeting refers to selective expression attained by the incorporation of transcriptional promoters with highly specific regulation of gene expression tailored to the cells of interest.

Examples of tissue-specific promoters include a liver-specific promoter (Zou et al., Endocrinology 138:1771-1774 (1997)); a small intestine-specific promoter (Oliveira et al., J. Biol. Chem. 271:31831-31838 (1996)); the promoter for creatine kinase, which has been used to direct of dystrophin cDNA expression in muscle and cardiac tissue (Cox et al., Nature 364:725-729 (1993)); and immunoglobulin heavy or light chain promoters for the expression of suicide genes in B cells (Maxwell et al., Cancer Res. 51:4299-4304 (1991)). An endothelial cell-specific regulatory region has also been characterized (Jahroudi et al., Mol. Cell, Biol. 14:999-1008 (1994)). Amphotrophic retroviral vectors have been constructed carrying a herpes simplex virus thymidine kinase gene under the control of either the albumin or alpha-fetoprotein promoters (Huber et al., Proc. Natl. Acad. Sci. U.S.A. 88:8039-8043 (1991)) to target cells of liver lineage and hepatoma cells, respectively. Such tissue-specific promoters can be used in retroviral vectors (Hartzoglou et al., J. Biol. Chem. 265:17285-17293 (1990)) and adenovirus vectors (Friedman et al., Mol. Cell. Biol. 6:3791-3797 (1986)) and still retain their tissue specificity.

Other elements siding specificity of expression in a tissue of interest can include secretion leader sequences, enhancers, nuclear localization signals, endosmolytic peptides, etc. Preferably, these elements are derived from the tissue of interest to aid specificity.

Viral vector systems useful in the practice of the instant invention include but are not limited to adenovirus, herpesvirus, adeno-associated virus, minute virus of mice (MVM), HIV, sindbis virus, and retroviruses such as Rous sarcoma virus, and MoMLV. Typically, the nucleic acid encoding the therapeutic polypeptide or peptide of interest is inserted into such vectors to allow packaging of the nucleic acid, typically with accompanying viral DNA, infection of a sensitive host cell, and expression of the polypeptide or peptide of interest.

For example, the DNA constructs of the invention can be linked through a polylysine moiety to asialo-oromucoid, which is a ligand for the asialoglycoprotein receptor of hepatocytes (Wu G.Y., and Wu, C.H., J. Biol. Chem. 263:14621-14624 (1988); WO 92/06180).

Similarly, viral envelopes used for packaging the recombinant constructs of the invention can be modified by the addition of receptor ligands or antibodies specific for a receptor to permit receptor-mediated endocytosis into specific cells (*e.g.*, WO 93/20221, WO 93/14188; WO 94/06923). In some embodiments of the invention, the DNA constructs of the invention are linked to viral proteins, such as adenovirus particles, to facilitate endocytosis (Curiel et al., Proc. Natl. Acad. Sci. U.S.A. 88:8850-8854 (1991)). In other embodiments, molecular conjugates of the instant invention can include microtubule inhibitors (WO 94/06922); synthetic peptides mimicking influenza virus hemagglutinin (Plank et al., J. Biol. Chem. 269:12918-12924 (1994)); and nuclear localization signals such as SV40 T antigen (WO 93/19768).

The nucleic acid can be introduced into the tissue of interest *in vivo* or *ex vivo* by a variety of methods. In some embodiments of the invention, the nucleic acid is introduced into cells by such methods as microinjection, calcium phosphate precipitation, liposome fusion, or biolistics. In further embodiments, the nucleic acid is taken up directly by the tissue of interest. In other embodiments, nucleic acid is packaged into a viral vector system to facilitate introduction into cells.

In some embodiments of the invention, the compositions of the invention are administered *ex vivo* to cells or tissues explanted from a patient, then returned to the patient. Examples of *ex vivo* administration of gene therapy constructs include Axteaga et al., Cancer Research 56(5):1098-1103 (1996); Nolta et al., Proc Natl. Acad. Sci. USA 93(6):2414-9 (1996); Koc et al., Seminars in Oncology 23 (1):46-65 (1996); Raper et al., Annals of Surgery 223(2):116-26 (1996); Dalesandro et al., J. Thorac. Cardi. Surg. 11(2):416-22 (1996); and Makarov et al., Proc. Natl. Acad. Sci. USA 93(1):402-6 (1996).

**Formulations and Pharmaceutical Compositions**

The peptides of the current invention can, for example, be synthesized or produced using recombinant methods and techniques known in the art. In a preferred embodiment the peptide can be fused to a carrier polypeptide/protein as for example the "maltose binding protein" (MBP) and produced using recombinant method. The term "recombinant protein" is used to indicate this construct. For example, fusion protein made from peptide of scorpion venom and MBP was used as antigens to successfully produce antibodies in rabbit (Legros, C. et al. Vaccine 20:934-42 (2001)). This shows that these fusion proteins can be successfully used as a vaccine providing efficient immune protection against *A. Australis* venom. Alternatively, the peptide of the present invention can be synthesized and then fused to a carrier molecule to improve its efficiency. In an alternative embodiment the peptides of the present invention or the recombinant protein comprising said peptide are pegylated. Pegylation is the conjugation of peptides or polypeptides with polyethylene glycol. Pegylated alpha interferon has used as a treatment for mice infected by the Venezuelan equine encephalitis virus (VEEV). The use of pegylated interferon results in greatly enhanced survival to infection to VEEV (Lukaszewski, R.A. and Brooks, T.J. J Virol 74:5006-15 (2000)). In human therapy, pegylated interferon is currently an efficient treatment of chronical infection with hepatitis C virus (Poynard, T., et al. Lancet 362:2095-100 (2003)).

The compositions of the invention will be formulated for administration through ways known in the art and acceptable for administration to a mammalian subject, preferably a human. In some embodiments of the invention, the compositions of the invention can be administered directly into a tissue by injection. In further embodiments of the invention the compositions of the invention are administered "locoregionally", *i.e.*, intravesically, intralesionally, and/or topically. In other embodiments of the invention, the compositions of the invention are administered systemically by injection, inhalation, suppository, transdermal delivery, etc. In further embodiments of the invention, the compositions are administered through catheters or other devices to allow access to a remote tissue of interest, such as an internal organ. The compositions of the invention can also be administered in depot type devices, implants, or encapsulated formulations to allow slow or sustained release of the compositions.

In order to administer therapeutic agents based on, or derived from, the present invention, it will be appreciated that suitable carriers, excipients, and other agents may be incorporated into the formulations to provide improved transfer, delivery, tolerance, and the like.

A multitude of appropriate formulations can be found in the formulary known to all pharmaceutical chemists: Remington's Pharmaceutical Sciences, (15th Edition, Mack Publishing Company, Easton, Pennsylvania (1975)), particularly Chapter 87, by Blaug, Seymour, therein. These formulations include for example, powders, pastes, ointments, jelly, waxes, oils, lipids, anhydrous absorption bases, oil-in-water or water-in-oil emulsions, emulsions carbowax (polyethylene glycols of a variety of molecular weights), semi-solid gels, and semi-solid mixtures containing carbowax.

Any of the foregoing formulations may be appropriate in treatments and therapies in accordance with the present invention, provided that the active agent in the formulation is not inactivated by the formulation and the formulation is physiologically compatible.

The quantities of active ingredient necessary for effective therapy will depend on many different factors, including means of administration, target site, physiological state of the patient, and other medicaments administered. Thus, treatment dosages should be titrated to optimize safety and efficacy. Typically, dosages used *in vitro* may provide useful guidance in the amounts useful for *in situ* administration of the active ingredients. Animal testing of effective doses for treatment of particular disorders will provide further predictive indication of human dosage. Various considerations are described, for example, in Goodman and Gilman's the Pharmacological Basis of Therapeutics, 7th Edition (1985), MacMillan Publishing Company, New York, and Remington's Pharmaceutical Sciences 18th Edition, (1990) Mack Publishing Co, Easton Penn. Methods for administration are discussed therein, including oral, intravenous, intraperitoneal, intramuscular, transdermal, nasal, iontophoretic administration, and the like.

The compositions of the invention may be administered in a variety of unit dosage forms depending on the method of administration. For example, unit dosage forms suitable for oral administration include solid dosage forms such as powder, tablets, pills, capsules, and dragees, and liquid dosage forms, such as elixirs, syrups, and suspensions. The active ingredients may also be administered parenterally in sterile liquid dosage forms. Gelatin capsules contain the active ingredient and as inactive ingredients powdered carriers, such as glucose, lactose, sucrose, mannitol, starch, cellulose or cellulose derivatives, magnesium stearate, stearic acid, sodium saccharin, talcum, magnesium carbonate and the like. Examples of additional inactive ingredients that may be added to provide desirable color, taste, stability, buffering capacity, dispersion or other known desirable features are red iron oxide, silica gel, sodium lauryl sulfate, titanium dioxide, edible white ink and the like. Similar diluents can be used to make compressed tablets. Both tablets and capsules can be manufactured as sustained release products to provide for continuous release of medication over a period of hours. Compressed tablets can be sugar-coated or film-coated to mask any unpleasant taste and protect the tablet from the atmosphere, or enteric-coated for selective disintegration in the gastrointestinal tract. Liquid dosage forms for oral administration can contain coloring and flavoring to increase patient acceptance.

The concentration of the compositions of the invention in the pharmaceutical formulations can vary widely, *i.e*., from less than about 0.1%, usually at or at least about 2% to as much as 20% to 50% or more by weight, and will be selected primarily by fluid volumes, viscosities, etc., in accordance with the particular mode of administration selected.

The compositions of the invention may also be administered via liposomes. Liposomes include emulsions, foams, micelles, insoluble monolayers, liquid crystals, phospholipid dispersions, lamellar layers and the like. In these preparations the composition of the invention to be delivered is incorporated as part of a liposome, alone or in conjunction with a molecule which binds to a desired target, such as antibody, or with other therapeutic or immunogenic compositions. Thus, liposomes either filled with or composed of a desired composition of the invention can be delivered systemically, or can be directed to a tissue of interest, where the liposomes then deliver the selected therapeutic/immunogenic peptide compositions.

Liposomes for use in the invention are formed from standard vesicle-forming lipids, which generally include neutral and negatively charged phospholipids and a sterol, such as cholesterol. The selection of lipids is generally guided by consideration of, *e.g.*, liposome size, acid lability and stability of the liposomes in the blood stream. A variety of lipids is described in, *e.g.*, Szoka et al. Ann. Rev. Biophys. Bioeng. 9:467 (1980); U.S. Patent NOs. 4,235,871; 4,501,728; 4,837,028 and 5,019,369, incorporated herein by reference.

A liposome suspension containing a composition of the invention may be administered intravenously, locally, topically, etc. in a dose which varies according to, *inter alia,* the manner of administration, the composition of the invention being delivered, and the stage of the disease being treated.

For solid compositions, conventional nontoxic solid carriers may be used which include, for example, pharmaceutical grades of mannitol, lactose, starch, magnesium stearate, sodium saccharin, talcum, cellulose, glucose, sucrose, magnesium carbonate, and the like. For oral administration, a pharmaceutically acceptable nontoxic composition is formed by incorporating any of the normally employed excipients, such as those carriers previously listed, and generally 10-95% of active ingredient, that is, one or more compositions of the invention, and more preferably at a concentration of 25%-75%.

For aerosol administration, the compositions of the invention are preferably supplied in finely divided form along with a surfactant and propellant. Typical percentages of compositions of the invention are 0.01%-20% by weight, preferably 1%-10%. The surfactant must, of course, be nontoxic, and preferably soluble in the propellant. Representative of such agents are the esters or partial esters of fatty acids containing from 6 to 22 carbon atoms, such as caproic, octanoic, lauric, palmitic, stearic, linoleic, linolenic, olesteric and oleic acids with an aliphatic polyhydric alcohol or its cyclic anhydride. Mixed esters, such as mixed or natural glycerides may be employed. The surfactant may constitute 0.1 %-20% by weight of the composition, preferably 0.25%-5%. The balance of the composition is ordinarily propellant. A carrier can also be included, as desired, as with, e.g., lecithin for intranasal delivery.

The compositions of the invention can additionally be delivered in a depot-type system, an encapsulated form, or an implant by techniques well known in the art. Similarly, the compositions can be delivered via a pump to a tissue of interest.

The compositions of the invention are typically administered to patients after the onset of symptoms, although treatment can also be prophylactic in some embodiments. Typically, treatment with direct administration of polypeptides is done daily, weekly, or monthly, for a period of time sufficient to reduce, prevent, or ameliorate symptoms. Treatment with the nucleic acids of the invention is typically done at intervals of several months. In some embodiments, administration of the compositions of the invention is done *in utero.*

The composition of the invention may also be provided in a kit as a slow-release composition such as a daily, weekly, monthly unit provided as a sponge, dermal patch, subcutaneous implant and the like in a wrapping or container as described above. In this case, the patient may release a unit of the composition from the container and applies it as indicated in the kit instructions. The composition may then be replaced at the end of the specified period by a fresh unit, and so on.

The present composition may also be administered by means of injection, as indicated above. Typically, the peptide may be administered by itself, or, for instance, in the case of a diabetic, in a composition also comprising insulin. The same applies for the slow-release forms of the composition. Similarly, the peptide of the invention may be administered in a composition that also comprises another drug. The proportion of peptides to the other drug(s) and carrier may be adjusted accordingly.

The levels of the delivered peptide to a patient may be monitored by immunoassay. To determine the level of the peptide of invention in blood following administration, *e.g.,* intramuscular or subcutaneous administration, an antibody assay may be performed with antibodies specific to the peptide sequence by any of the protocols known in the art. Polyclonal or monoclonal antibodies may be utilized. The level of the peptide in blood may then be correlated with the progress of the inhibition of any of the diseases the patient is afflicted with.

**Examples**

1. ***In silica* design:**

**1.1. Tridimensional construction of peptides.**

The construction of the helical peptides ApoE WT and Aβ 29-42 was carried out using Hyperchem (release 6.1 for windows-Hypercube) assigning Φψ angles of -58° and -47° corresponding to the classical α-helical structure. This conformation was assumed as in Brasseur *et al.* (Brasseur, R., Lins, L., Vanloo, B, Ruysschaert, J. M. and Rosseneu, M. (1992) Proteins 13(3):246-257). The conformation of the backbone and the side chains was optimized by a steepest descent procedure completed by a conjugated gradient procedure.

**1.2. Molecular modelling of the ApoE WT/Aß interaction.**

The hypermatrix procedure, derived from the method allowing surrounding a drug with lipids (Brasseur, R., Goormaghtigh, E., and Ruysschaert, J. M. (1981) Biochem. Biophys. Res. Commun. 103, 301-310), was used to carry out the complexes between ApoE WT and Aβ 29-42. In this method, the Aß helix is maintained in a fixed position while the ApoE WT α helix moves around with five degrees of freedom (including 36 rotations around Aß and 36 self-rotations by steps of 10°, 10 translations along the x- and z-axes by steps of 1 and 0,5 A, respectively, and 20 slopes of 1° as compared to the Aß helix axis) in order to explore a huge number of relative positions (2,6.10⁶ positions). In the initial configuration both helices are antiparallel. The interaction examined comprises Coulomb, van der Waals and solvation components. For each relative position, the energy of interaction is calculated as the sum of the Coulomb, van der Waals and solvation energies. The [Aβ 29-42/ ApoE WT] complex of lowest energy is retained.

Those results, which confirm those of Lins *et al*. (Lins, L., Thomas-Soumarmon, A., Pillot, T., Vandekerchkhove, J., Rosseneu, M., and Brasseur, R. (1999) J. Neurochem. 73, 758-769), clearly indicate that the most hydrophobic face of ApoE WT interacts with the Aβ 29-42 helix.

Moreover, the two helices are parallel ensuring an optimal contact surface between both peptides.

The Molecular Hydrophobicity Potential (MHP) of ApoE WT based on atomic transfert energy was calculated as previously described in Brasseur, R. (1991) J. Biol. Chem. 266, 16120-16127. Calculations show that the most hydrophobic part of ApoE WT is involved in the interaction.

**1.3. Determination of key residues in the interaction with ApoE** wild type.

The complex of lower energy selected by the hypermatrix procedures was optimized using the angular dynamics method previously described (Brasseur, R. (1995) J. Mol. Graph. 13, 312-322). Then, the PEX algorithm (Thomas, A., Bouffioux, O., Geeurickx, D., and Brasseur, R. (2001) Proteins 43, 28-36) was used in order to determine the most important residues of the ApoE WT helix involved in the interaction with Aß in the [Aβ 29-42/ ApoE WT] complex.

The accessible surface (ASA) of each ApoE WT residue, the residues in interaction, the distance between residues and the energy of interaction were calculated. The calculation of ASA was carried out using the method of Shrake and Rupley with 642 points (Shrake A. and Ruppley (1973) J.A. J. Mol. Biol. 79: 351-371).

The PEX calculations were performed from the PDB file of the [Aβ 29-42/ ApoE WT] complex generated by the hypermatrix procedure.

Several residues that could be mutated in the ApoE WT peptide to improve the ApoE WT/Aβ 29-42 interaction were defined.

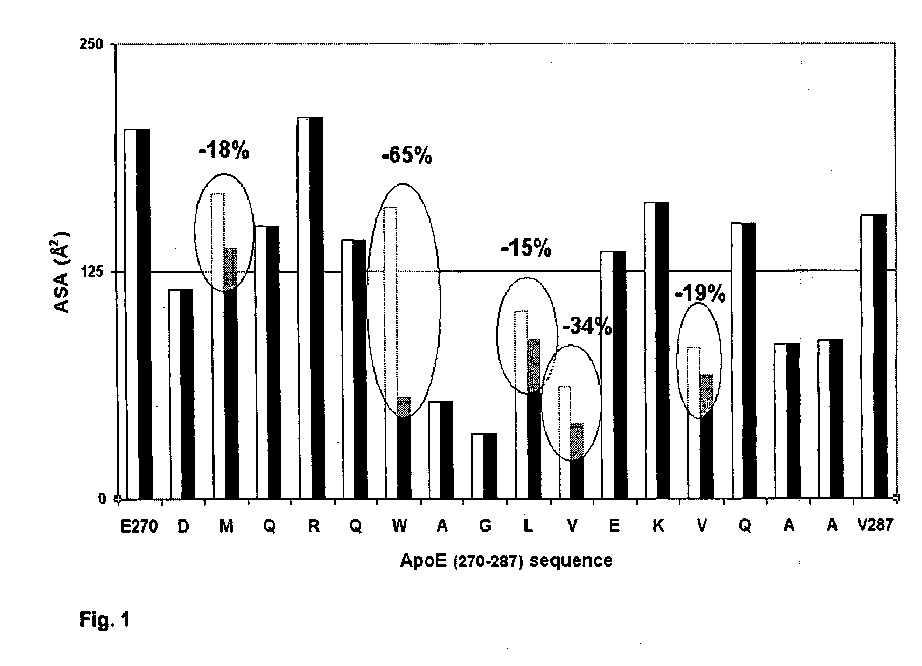

Figure 1 represents the results of the PEX analysis, expressed in Accessible Surface Area (ASA) as a function of ApoE WT residues in the [Aβ 29-42/ ApoE WT] complex and alone (in Å²). Circles highlight residues with the highest ASA decrease (in %) (■ (closed rectangle) = ApoE WT in the complex, □ (open rectangle) = ApoE WT alone).

It is likely that the residues of ApoE WT which lost accessible surface in the complex as compared to the ApoE WT fragment alone are involved in the interaction. The residues that are located on the same side of the ApoE WT helix are: M272, W276, L279, V280 and V283, which lost 18%, 65%, 15%, 34% and 19% of their accessible surface, respectively. In order to improve the interaction, these positions were selected for mutations purposes.

Two criteria were used to define the mutations. First, the substituting residues must be hydrophobic because of the nature of the interaction highlighted in Lins *et al*. (Lins, L., Thomas-Soumarmon, A., Pillot, T., Vandekerchkhove, J., Rosseneu, M., and Brasseur, R. (1999) J. Neurochem. 73, 758-769). Second, they must have a high helicity propensity since ApoE WT amphipathic fragments are defined as helical (Brasseur, R., Lins, L., Vanloo, B, Ruysschaert, J.M. and Rosseneu, M. (1992) Proteins 13(3):246-257). Based on these criteria, M, W, L and V were chosen for the substitutions.

**1.4. Generation of ApoE mutants and selection of [Aβ 29-42/ ApoE mutant n] complex.**

**1.4.1. Generation of ApoE mutants.**

To generate mutants of the ApoE270-287 peptide, the present inventors used the complex of ApoE270-287 and Aß29-42 helix structures obtained after the hypermatrix procedure as a template. This complex was loose enough to allow residue substitutions without generating harsh steric clashes. All key positions amino acids of the wild type ApoE peptide in interaction with Aß in the complex were open to substitutions generating 1024 possible peptides by random combination of substitutions.

The energy of each mutant/Aß complex was minimized by an angular Monte Carlo procedure based on the angular dynamics previously described (Brasseur, 1995). This procedure differs from a current energy minimization on several points: valence angles and bond lengths are maintained constant, atom movements are rotations around molecule torsion axes and rotation movements are propagated along the chain; finally, the energy of atomic interactions is distributed on rotation axes, and every axis is independently minimized resulting in a local rather than a global energy minimization. During the procedure, one molecule remains still and the other moves around and along. The two molecules move their side chains and the mutant also adapts its backbone architecture. The energy of interaction is minimized at 25°C for 200 steps of Monte Carlo procedure, each step allowing a maximal 3D translation of 0.25 Å of the moving molecule centre and a maximal tilt of 1° of its axis. At each step, 7 successive rotations of all axes at 25°C optimized the side chain structures. The energy of the system is the sum of intra and intermolecular energies of non-bound interactions: for the intramolecular interaction Van der Waals (using Levitt description of soft-atom with 1000 kcal/mol as a limit energy value (Levitt, 1983) and electrostatic energy (Coulomb) were summed; for the intermolecular interactions, Van der Waals (Levitt description of soft-atom with 100 kcal as a limit energy value), Coulomb (with a sigmoid description of ε variation and FCPAC atomic charges (Thomas *et al.,* 2004)) and two terms of hydrophobicity (*i.e*., due to peptide-peptide interactions and to water/peptide interaction (Brasseur, 1995)) were calculated. The first selection step of peptide/Aß29-42 complexes is at that point on minimal total energy (internal and external atomic interactions).

**1.4.2. Complex Analysis and final selection**

Complexes were characterized by their energy patterns: the residue Mean Force potential was calculated from atomic mean force potential scale (Melo and Feytmans, 1997). Scale for atomic Mean Force Potential values (MFP) was prepared by computing all atomic interactions in of 500 3D structures (Word *et al*., 1999). The equation of Lenard Jones was used for Van der Waals energy term. A sigmoid description of the dielectric constant and the FCPAC partial atomic charges (Thomas *et al.,* 2004) were used for the Coulomb energy. Last, the equation developed by Brasseur (1995) for inter and molecule/solvent hydrophobicity was used. All energy values are in Kcal/mol.

**1.4.3. Calculation of the template complex**

The present inventors started from the molecular modelling approach by Lins *et al*. (1999) and used the 270-287 fragment of apolipoprotein E3 as template for the design of an anti-Aβ peptide. In a first step, the inventors calculated the best ApoE270-287 (EDMQRQWAGLVEKVQAAV) (SEQ ID NO: 2) and Aß 29-42 (GAIIGLMVGGVVIA) (SEQ ID NO: 1) complex using a hypermatrix procedure. 2.6.10⁶ relative positions were explored and the structure with the lowest energy was saved. The orientation of the two helices is antiparallel as previously reported (Lins *et al*., 1999). The role of hydrophobicity is primordial in the formation of the complex as seen by comparing hydrophobic ASA of residues in the free peptides and in the complex. Hydrophobic ASA of the complex (1,760 Å²) is 21% lower than the sum of hydrophobic ASA of the free peptides (2,229 Å²). In parallel, the hydrophilic ASA is almost unchanged in the complex as compared to the free peptides. This indicates that formation of the complex hides the hydrophobicity of isolated molecules. Those results confirm those of Lins *et al.* (1999) and clearly point towards hydrophobicity as a key parameter for the complex stability.

**1.4.4. Identification of key residues.**

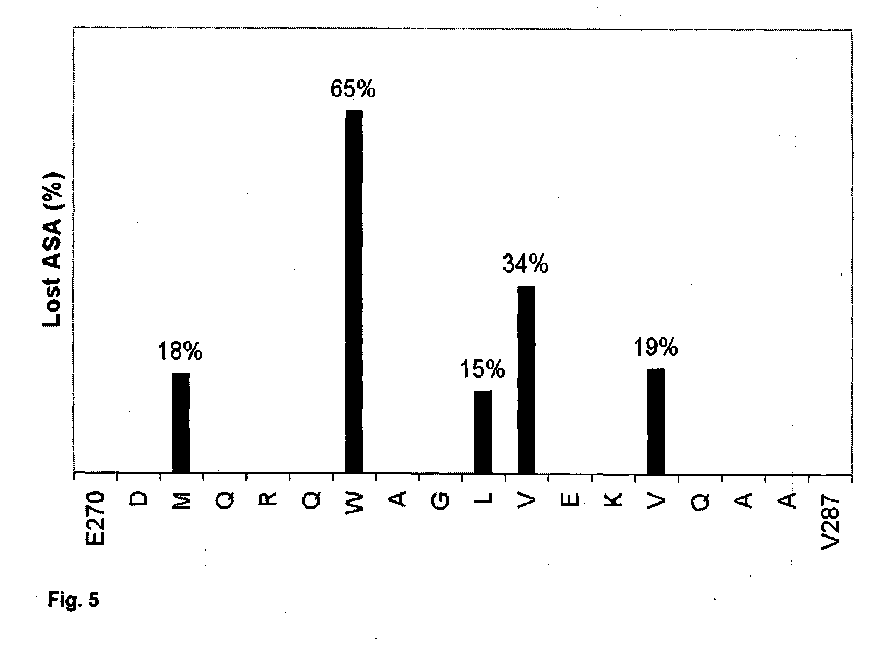

The ApoE270-287 residues specifically involved in the interaction with Aβ 29-42 were characterized by analysis of accessible surface (Figure 5). It was assumed that peptide residues of ApoE270-287 with less water-accessible surface in the complex than in the free form were implicated in the interaction. Those residues are located on the same side of the ApoE helix: M272, W276, L279, V280 and V283 and they have lost 18%, 65%, 15%, 34% and 19% of their solvent accessibility in the complex, respectively. These residues were selected for mutation by substitution.

**1.4.5. Residues proposed for substitution.**

Two criteria were used to define which residues would be used for substitution. First, in the template complex, most residues are involved in apolar interactions, hence residues proposed for substitutions had to be hydrophobic to keep the initial type of interaction. Second, the ApoE structure is an amphipathic helix, hence substitutes had to have a high helix propensity (Brasseur *et al*., 1992). Based on these criteria, Met (M), Trp (W) and Leu (L) were chosen. Val (V) was also selected due to its high hydrophobicity and its presence in the original peptide.

**1.4.6. Mutant complex calculations and initial selection:**

Using the original ApoE-Aß complex, 1024 structures obtained were tested by combinations of residue substitution for M272, W276, L279, V280 and V283. All peptides were energy-minimized in interaction with Aß 29-42 by 1400 steps of a Monte Carlo procedure based on angular dynamics. A primary selection was based on the minimization of the energy of intra plus intermolecular interactions.

Six complexes were selected, which all have gained in global energy as compared to the initial ApoE-Aß complex. Analysis of their sequences (Table 1) shows that Met 3 of the original ApoE peptide is frequently conserved, probably because of its side chain flexibility, which allows a high capacity for interaction. Conversely, Trp residue seems to be excluded from central positions 7 and 10. Residues at the other positions are more variable.

**1.4.7. Final selection of peptides**

In the previous calculation procedures, valence angles and bond lengths were constant. At that step, peptide geometry of complexes is relaxed by a conjugate gradient procedure using HyperChem that converged to 0.1 Kcal/step. A close analysis of the 6 relaxed complexes was then carried out based on the analysis of the partner interactions (Aß and the mutant peptide) (Tables 2 and 3). If 6 complexes have a global gain of energy with respect to the ApoE-Aß complex, only 3 have gained in their energy of peptide interaction (Table 2). These 3 complexes are with peptides 11 (SEQ ID NO: 3) "complex 11", peptide 12 (SEQ ID NO: 4) "complex 12", and peptide 413 (SEQ ID NO: 7) "complex 413", and were thus selected for further analysis.

From the three complexes, two types can be identified according to the term of the energy gain, either electrostatic (complexes 12 and 413) or hydrophobic (complex 11).

Complex 11 gains 5 kCal/mol in Epho (bilayer hydrophobicity) inter as compared to the ApoE-Aß complex and shows a significant gain in Mean Force Potential supporting that several atoms have found favourable partners. The Aß surface initially covered by ApoE is increased by 133 Å² further supporting a good Aß-peptide 11 surface matching (Table 2).

The main interesting feature of complexes with peptides 413 and 12 resides in their electrostatic gains and a good matching of the two peptides surface (56 Å² more Aß surface is covered by peptide 12 as compared to ApoE WT, and 31 Å² more is covered by peptide 413) (Table 2). The two peptides have kinked their backbone to enable the interaction of the N-terminal end of Aß with the C-terminal end of the two peptides.

Reasons for pairing improvement are further analysed in Table 3. In this table the closest inter-peptide atomic interactions of residues in the complexes are listed, the first three interactions for the initial ApoE-Aß complex only, the first one for peptides 11 and 413 in complex with Aß. This highlights the major role of W276 in the initial ApoE-Aß complex. Tryptophan is a bulky residue, hence its role in binding is easy to understand. Its central position in the ApoE peptide keeps the Aß helix away, decreasing the other residue binding possibilities. Mutation of this tryptophan, which was frequently noticed in our peptides, increases the peptide flexibility (backbone kinking is observed) and enables a more complete surface pairing. Hence the terminal valine (V18) of the peptides (corresponding to V289 in the ApoE) becomes a major partner of Aß as do residues at positions 3 and 7.

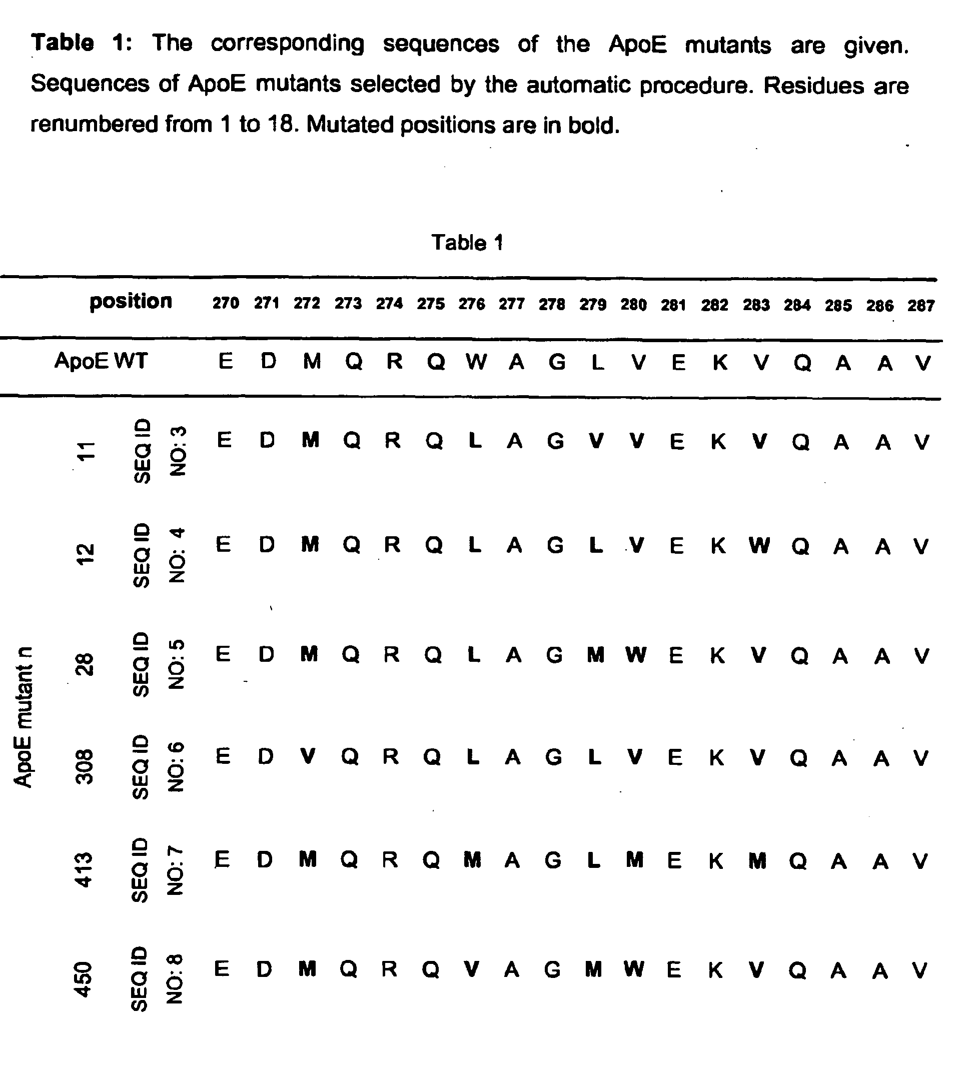

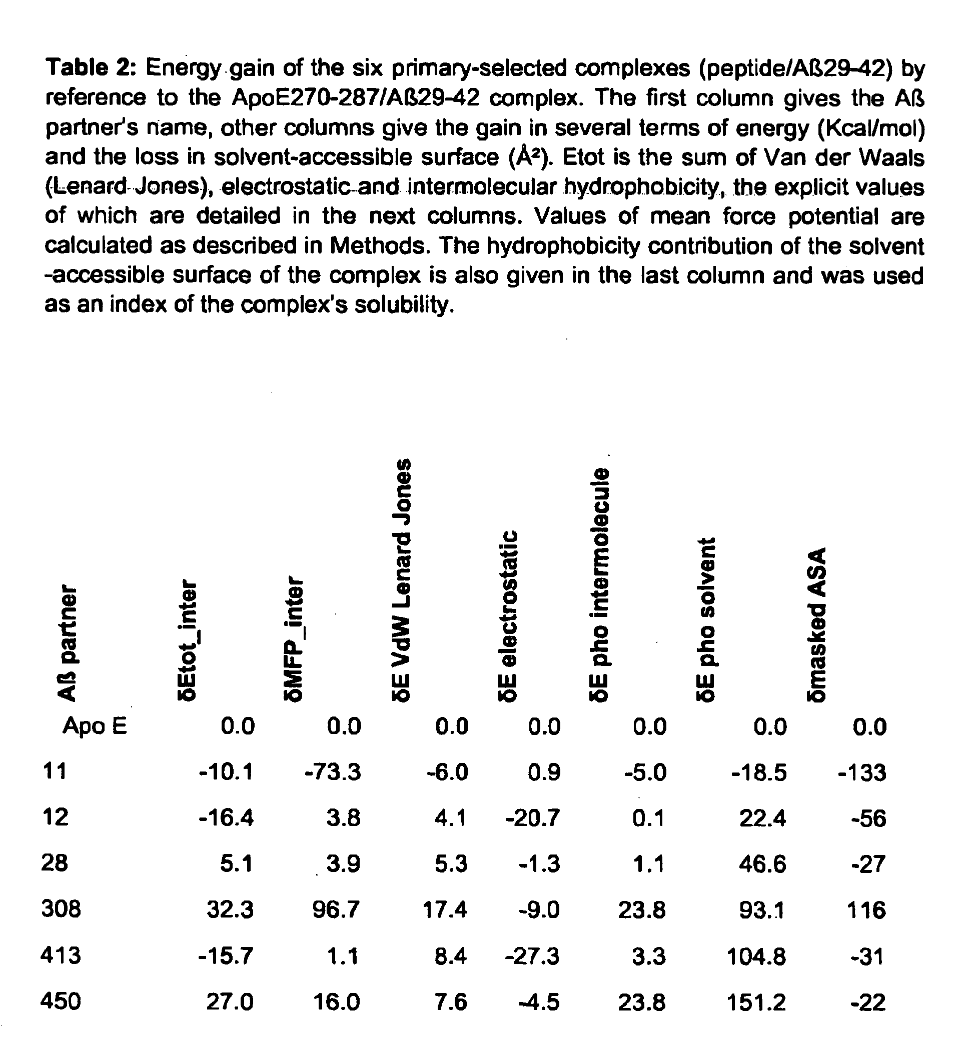

- The following table 3 summarizes the Pex analysis of complexes. In the first column, the Aß residues are listed and, in the following columns, individual atomic interactions are described with: the atom centre-to-centre distance, the name of the Aß and partner atoms, the number and name of the partner residue. The three shortest interactions are listed for the Aß complex with ApoE, the shortest one for the Aß complex with peptide 11 and 413, respectively.

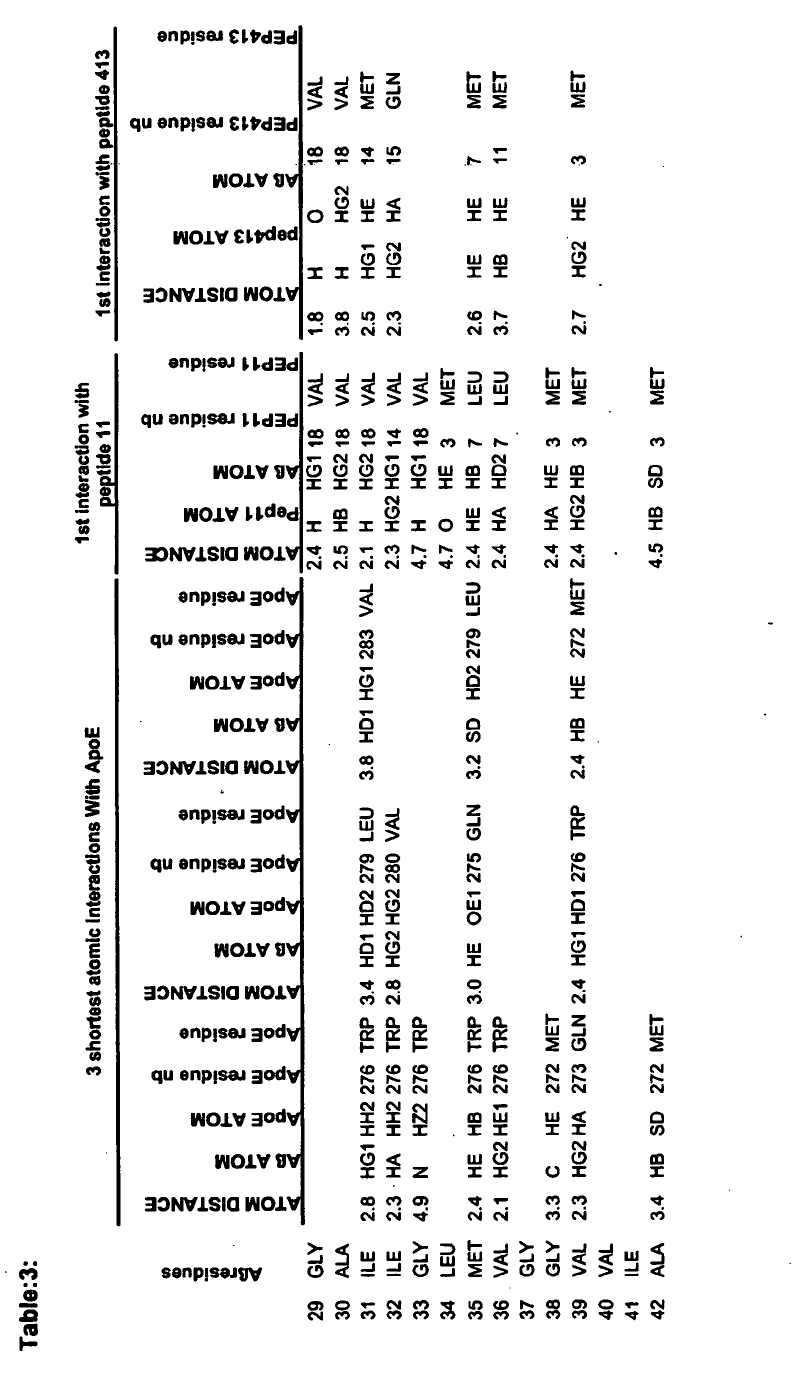

**Table 4.: Lipid fusion and leakage induced by Aß 29-42 and inhibitory effects of ApoE WT, ApoE mutant 11 and ApoE mutant 413 monitored by fluorescence at room temperature at a 1/1 peptide ratio. Values are in % and are averages of values in the plateau of fluorescence (between 10 and 15 minutes) of three different experiments.**

| Peptides added to the liposomes | % of fusion after 15 minutes | % of leakage after 15 minutes |
|---|---|---|
| Aß alone | 100% | 28% |
| Aß + mutant 413 (R=1) | 21% | 4% |
| Aß + mutant 11 (R=1) | 17% | 0% |
| Aß + ApoE WT (R=1) | 89% | ND |

**2. Synthesis of peptides**

Peptides 11 and 413 were synthesized according to a classical method. They were all C-terminal and N-terminal blocked (N-amidated and C-acetylated) and had a purity of 80% for Aß 29-42 peptide and 95% for ApoE peptides.

**3. In vitro assays:**

**3.1. Preparation of SUV**

The experimental part was carried out on small unilamellar vesicles (SUV). They were made of phosphatidylcholine (PC), phosphatidylethanolamine (PE), phosphatidylinositol (PI), phosphatidylserine (PS) sphingomyelin (SM) and cholesterol (Chol) (30%:30%:2,5%:10%:5 %:22,5% respectively; w/w).

All solvents came from Sigma (St Louis, USA), lipids were from Lipid Product (Surrey, UK), Sigma (St Louis, USA) and Aventis Polar lipids (Alabaster, USA). We used a sonicator from Sigma Chemical (USA) and the spectrofluorimeter LS-50B was from Perkin-Elmer (Nolwalk, USA).

The liposomes were prepared by dissolving the lipids in chloroform/methanol (2/1 vol/vol). After evaporation, the film was dried for 2 hours before being rehydrated with a Tris buffer pH 7,4 (Tris-HCI 10mM, NaCl 150mM, EDTA 0,5% and NaN₃1mM), then incubated at 37°C with stirring every 10 minutes. The solution was sonicated at 50W twice for 5 minutes. The particulate matter and the residual Multi Lamellar Vesicles were discarded by a 5 minutes centrifugation at 2000g. The phospholipid concentration was determined by the method of Barlett (Barlett et al., (1959) J. Biol. Chem. 65, 2146-2156).

**3.2. Fusion of lipid phase.**

Lipid fusion is monitored by fluorescence measurement using the method previously described by Hoekstra (Hoekstra, D. and Klappe, K. (1986) Biosci. Rep. 6, 953-960). In this method, a R-18 (octadecyl rhodamine B chloride)-labelled population of SUV is mixed to unlabelled liposomes.

In the presence of a fusogenic agent, an increase in the fluorescence signal is observed due to the dilution of the R18 in the lipid phase.

Labelled and unlabelled liposomes (1:4 w/w) and Aß 29-42 peptide (at different peptide/lipid molar ratio between 0,01 and 0,2) were mixed at room temperature and the fluorescence signal was recorded (excitation wavelength at 560 nm and emission wavelength at 590 nm). To avoid distortion of resulting fluorescence curves due to the buffer, its signal was measured in the same experimental conditions.

It was previously shown that Aß 29-42 induces *in vitro* liposome fusion (Mingeot-Leclercq M.P. et al., (2002) Chemistry and Physics of Lipids 120, 57-74). Dequenching of R18 fluorescence, a lipophilic probe allows monitoring of lipid fusion. In this study, this method was used to measure the inhibitory effect of ApoE WT and mutants 11 and 413 on the liposome fusion induced by Aß 29-42.

Neither the ApoE WT fragment nor its mutants have fusogenic properties: none of them induces lipid fusion (data not shown).

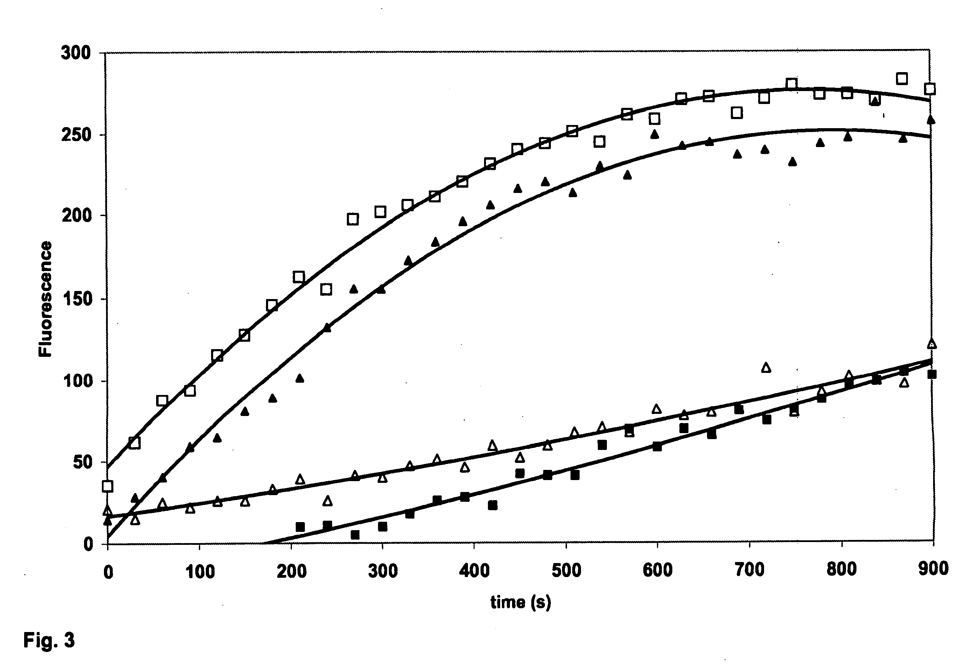

Figure 3 shows lipid fusion induced by Aβ 29-42 peptide and the inhibitory effect of the addition of ApoE WT, ApoE mutant 11, and ApoE mutant 413.

In figure 3, R18-labelled and unlabelled SUV (PC 30%, PE 30%, PI 2,5%, PS 10% SM 5% and Chol 22,5%) in buffer. □ (open rectangle) = Aβ 29-42 ; ▲ (closed triangle) = [Aβ 29-42/ ApoE WT] complex (molar ratio=1); A (open triangle) = [Aβ 29-42/ ApoE mutant 413] complex (molar ratio=1); ■ (closed rectangle) = [Aβ 29-42/ ApoE mutant 11] complex (molar ratio=1).

In the absence of the Aβ 29-42 peptide (only liposomes with buffer), no significant increase of the fluorescence signal was observed (data not shown). By contrast, in presence of the Aβ 29-42 peptide (peptide/lipid ratio of 0,4 (mol/mol)), the fluorescence rapidly increases, indicating lipid destabilisation and fusion of the two liposome populations. This measure was taken as the 100% fusion reference for the graph of figure 3. When the ApoE WT peptides were added to the Aβ 29-42 fragment at a molar ratio of 1, a decrease in fluorescence signal (to about 91%) is observed, indicating that the interaction between both peptides decreases the fusogenic potential of Aβ 29-42. When mutants 11 and 413 were added, we observed that the fluorescence signal was significantly weaker (to 23% and 25%, respectively).

. For these assays, a 1/1 molar ratio was used, in agreement with the results of the calculations made and those of Lins *et al.* (Lins, L., Thomas-Soumarmon, A., Pillot, T., Vandekerchkhove, J., Rosseneu, M., and Brasseur, R. (1999) J. Neurochem. 73, 758-769).

The interaction between each mutant (11 and 413) and Aβ 29-42 is optimised as compared to the ApoE WT peptide, in agreement with the calculations made.

**3.3. Leakage of liposomes**

As shown above, Aβ 29-42 induces an increase of R18 fluorescence and mutants 11 and 413 decrease this fluorescence. By leakage, it was verified that this effect is due to a true perturbation of the membranes and not only to a dilution of R18 which would occur by vesicle aggregation.

In this leakage assay, performed according to the procedure described in Ellens *et al.* (Ellens, H., Bentz, J., and Szoka, F. C. (1985) Biochemistry 24, 3099-3106), a substance encapsulated in the liposomes is followed by fluorescence measurement. HPTS (8-aminonaphtalene-1, 3, 6-trisulfonic acid) and his quencher DPX (p-xylylenebis[pyridinium] bromide) were both encapsulated in the aqueous phase of the same liposomes. Liposomes were eluted on a Sephadex G75 column to remove the excess of HPTS and DPX.

Fluorescence was measured at room temperature using excitation and emission wavelengths of 450 nm and 512 nm, respectively. Leakage induced by the Aβ peptide was followed by measuring the dequenching of HPTS released into the medium. The percentage of release induced by the peptide was defined as Ft/Ftot x100 , where Ft is the fluorescence signal measured at time t and Ftot is the signal obtained after lysing the vesicles with 0,5% Triton X100.

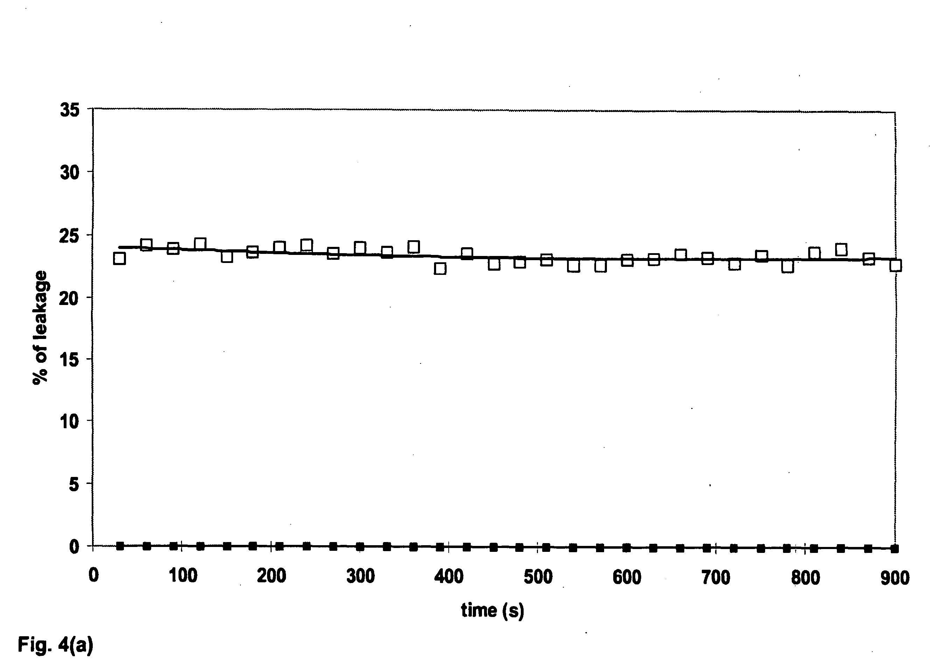

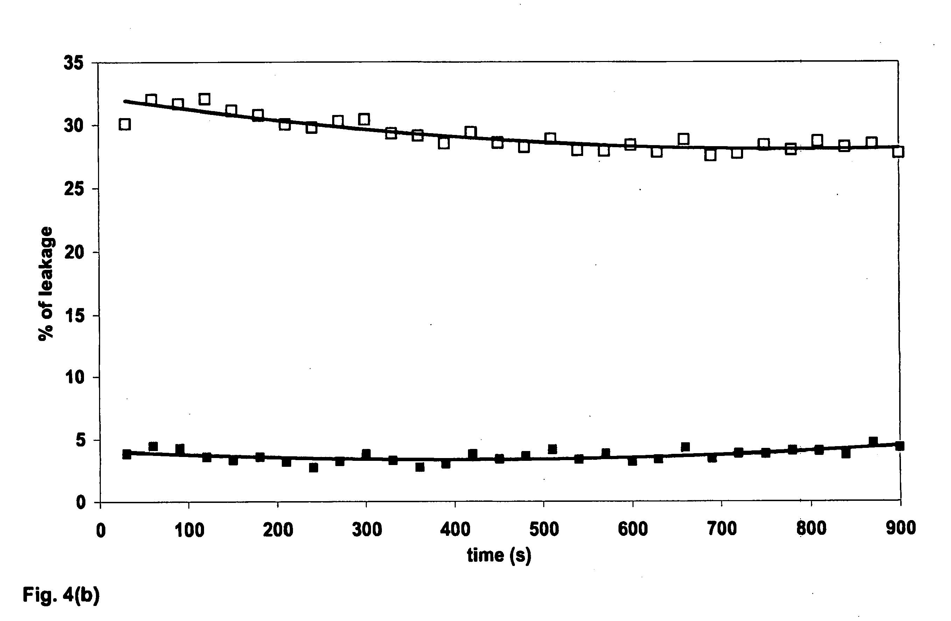

In order to assess membrane destabilization of SUV induced by the Aβ 29-42 peptide and to confirm the inhibitory effect of mutants 11 and 413 on this destabilization, HTPS and DPX were encapsulated in liposomes. It was first checked that mutants 11 and 413 have no destabilization effects on the membranes (data not shown). In presence of Aβ 29-42 (peptide/lipid molar ratio of 0,4), the fluorescence of HTPS immediately increases, indicating that membrane destabilisation occurs (figures 4a and b). In contrast, when mutants 11 and 413 were added, the signal is much weaker independently of the mutant/Aβ 29-42 molar ratio used, indicating that both mutants have inhibitory effect on the destabilization properties of Aβ 29-42 (figures 4a and b). Those results are in agreement with the lipid fusion assays and calculations.

**4. Inhibitory effect of the ApoE 270-287 peptide onto the toxicity induced by the Aβ 1-42 peptide**

In order to test the inhibitory effect of the ApoE 270-287 peptides onto the toxicity induced by the Aβ1-42 peptide, cell toxicity assays were performed *in vitro* in presence of the Aβ 1-42 and ApoE 270-287 (WT) peptides.
- Cell toxicity assay of Aβ 1-42 on human neuroblastoma cell line.
- Optimization of the ApoE 270-287 concentration to be used.
- Cell toxicity assay in presence of both the Aβ 1-42 and ApoE 270-287 peptides.

**4.1 Cell toxicity assay of Aβ 1-42 on human neuroblastoma cell line**

Initially, we performed cell toxicity assays with several Aβ peptides, namely, Aβ 1-42, Aβ 1-40, and Aβ 25-35 peptides, in order to test their respective toxicity. Indeed, it is known that the Aβ 1-40 peptide does not induce cell toxicity, while the Aβ 25-35 peptide induces cell toxicity through a different mechanism than the Aβ 1-42 peptide.

The inventors tested several peptide concentrations to determine the LC50, using the MTS assay (Dupiereux et al., (2005) Biochem. Biophys. Res. Commun. 331 (4):894-901), (MTS= (3-(4,5-dimethylthiazol-2-yl)-5-(3-carboxymethoxyphenyl)-2-(4-sulfophenyl)-2H-tetrazolium, inner salt), which measures the mitochondrial and cytoplasmic dehydrogenase activity of the cells. The cell line used was derived from human neuroblastoma (SH-SY5Y).

The assay is performed in 96-wells plates on 30000 cells / well after a 24h incubation with the Aβ 1-42 peptide.

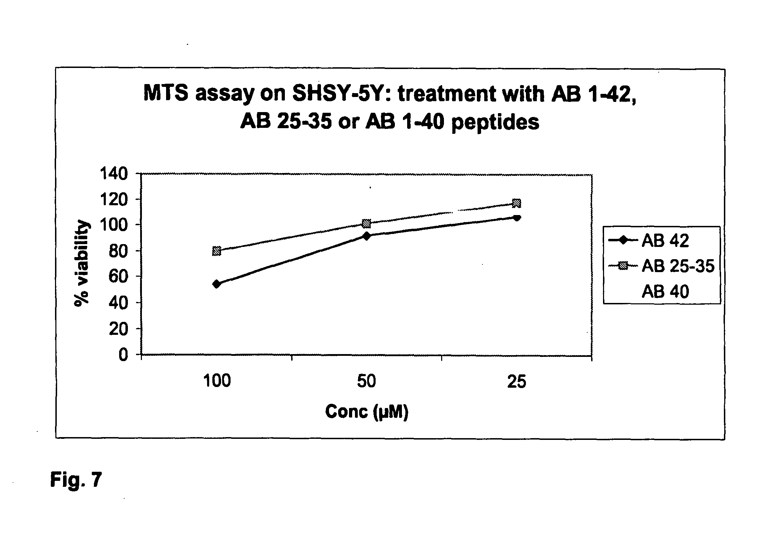

Results are shown in figure 7 wherein the percent viability is plotted against increasing peptide concentrations. It appears that the Aβ 1-42 peptide induces cell toxicity in a dose-dependent fashion, while the Aβ1-40 peptide does not induce any. The Aβ25-35 peptide also induces cell toxicity but to a lesser extend.

According to these results, a 100µM concentration of the Aβ 1-42 peptide, which gives 50% viability, will be used to test the inhibitory effect of the ApoE 270-287 peptide on cell toxicity induced by the Aβ 1-42 peptide. However, it is first necessary to determine a working concentration for the ApoE 270-287 peptide that does not itself induce cell toxicity.

**4.2. Optimization of the ApoE 270-287 peptide concentration to be used**

Cells are derived from human neuroblastoma (SH-SY5Y) and were assayed using the MTS assay, as described above. Experiments were performed on 30,000 cells/well in 96-wells plates. ApoE 270-287 peptide concentrations ranging from 100 to 2 µM were tested.

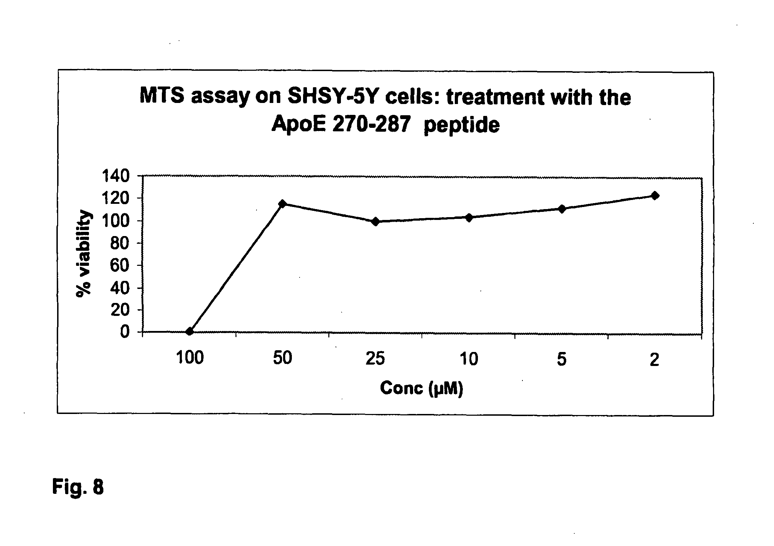

Results shown in figure 8 indicate that the ApoE 270-287 peptide induces cell toxicity only at 100µM, with a sharp drop in cell viability. Therefore, peptide concentrations lower than 100µM will be used.

**4.3. Cell toxicity assay in presence of both the Aβ 1-42 and ApoE 270-287 peptides**

Once optimal concentrations of both the Aβ 1-42 and ApoE 270-287 peptides were determined, we proceeded to test the inhibitory effect of the ApoE 270-287 peptide on cell toxicity induced by the Aβ 1-42 peptide. To that effect, cells were incubated in presence of 2µM ApoE 270-287 and 100µM Aβ 1-42 (ratio 1/50).

This ratio was determined through membrane fusion inhibition experiments on liposomes, whose lipid composition is very similar to neural cells membrane (see, paragraph 3.2). The ApoE 270-287 concentration used is the one inhibiting liposome membrane fusion induced by the Aβ 1-42 peptide. Experiments were performed as described above. SH-SY5Y cells were incubated for 24h with 2µM ApoE and 100µM Aβ 1-42.

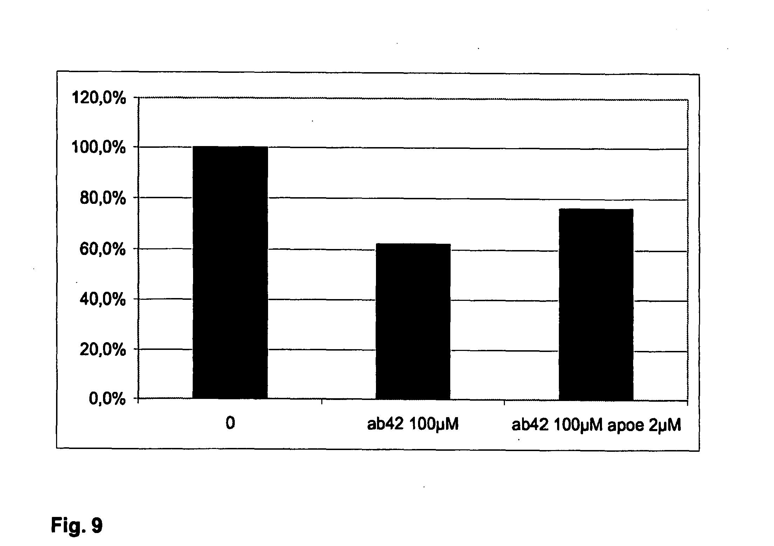

Results shown in figure 9 indicate that the toxicity induced by 100µM of Aβ 1-42 peptide (60% viability) is slightly decreased in presence of 2µM of ApoE 270-287 peptide (80% viability). Therefore, the complementary peptide ApoE 270-287 is able to counteract the toxicity induced by the Aβ 1-42 peptide *in vitro.*

These results confirm that using complementary peptides to counteract the cytotoxicity induced by the Aβ peptide is an interesting strategy and constitutes a valid approach towards a therapy for Alzheimer's disease.

## Claims

1. An isolated peptide derived from the wild type ApolipoproteinE 3 peptidic fragment 270-287 (ApoE WT) of sequence EDMQRQWAGLVEKVQAAV (SEQ ID NO: 2), wherein the peptide is from 18 to 50 amino acid residues in length and having improved interaction with the β-Amyloid peptidic fragment 29-42 (Aβ 29-42) GAIIGLMVGGWIA (SEQ ID NO: 1), said peptide comprising one or a combination of amino acid sequences selected from the group consisting of:
-EDMQRQLAGWEKVQAAV (SEQ ID NO: 3)
-EDMQRQLAGLVEKWQAAV (SEQ ID NO: 4)
-EDMQRQLAGMWEKVQAAV (SEQ ID NO: 5)
-EDVQRQLAGLVEKVQAAV (SEQ ID NO: 6)
-EDMQRQMAGLMEKMQAAV (SEQ ID NO: 7) and
-EDMQRQVAGMWEKVQAAV (SEQ ID NO: 8)
-or a multimer thereof, or a variant thereof wherein 1 or 2 amino acid residues is/are deleted from the N- and/or C-terminus in SEQ ID NOs: 3, 4, 5, 6, 7 and 8.

2. The peptide according to claim 1, which peptide when complexed to the β-Amyloid peptidic fragment 29-42 (Aβ 29-42) GAIIGLMVGGVVIA (SEQ ID NO: 1), decreases the fusogenic activity of Aβ 29-42.

3. The peptide according to claim 1 or 2, wherein said peptide comprises an amino acid sequence selected from the group consisting of:
EDMQRQLAGWEKVQAAV (SEQ ID NO: 3) and
EDMQRQMAGLMEKMQAAV (SEQ ID NO: 7)
or a multimer thereof.

4. A recombinant protein, polypeptide or oligopeptide comprising one or a combination of the peptides according to any one of claims 1 to 3

5. A polynucleotide encoding any one of the peptide of claim 1 to 3 or encoding the recombinant protein, polypeptide or oligopeptide of claim 4.

6. A detection method of the β-Amyloid protein or its peptidic fragment β-Amyloid 29-42 (Aβ 29-42) GAIIGLMVGGVVIA (SEQ ID NO: 1), wherein said method comprises:
- a step of contacting a biological sample containing the β-Amyloid protein or its peptidic fragment β-Amyloid 29-42 (Aβ 29-42) with a peptide or recombinant protein comprising one or a combination of amino acid sequences selected from the group consisting of SEQ ID NOs 3, 4, 5, 6, 7, and 8, or a multimer thereof; or a variant thereof wherein 1 or 2 amino acid residues is/are deleted from the N- and/or C-terminus in SEQ ID NOs: 3, 4, 5, 6, 7 and 8, and
- a step of detecting the binding of said peptide or of said recombinant protein to the β0-Amyloid protein or to its β-Amyloid peptidic fragment 29-42 (Aβ 29-42).

7. A kit for use in the detection of the β-Amyloid protein or its peptidic fragment β-Amyloid 29-42 (Aβ 29-42) GAIIGLMVGGVVIA (SEQ ID NO: 1), the kit comprising a peptide or recombinant protein comprising one or a combination of amino acid sequences selected from the group consisting of SEQ ID NOs 3, 4, 5, 6, 7, and 8, or a multimer thereof; or a variant thereof wherein 1 or 2 amino acid residues is/are deleted from the N- and/or C-terminus in SEQ ID NOs: 3, 4, 5, 6, 7 and 8, the kit further comprising an antibody for detecting the binding of said peptide or of said recombinant protein to the β-Amyloid protein or to its β-Amyloid peptidic fragment 29-42 (Aβ 29-42).

8. A binding assay for the detection of the β-Amyloid protein or its peptidic fragment β-Amyloid 29-42 (Aβ 29-42) GAIIGLMVGGVVIA (SEQ ID NO: 1), the assay involving the use of a peptide or recombinant protein comprising one or a combination of amino acid sequences selected from the group consisting of SEQ ID NOs 3, 4, 5, 6, 7, and 8, or a multimer thereof, or a variant thereof wherein 1 or 2 amino acid residues is/are deleted from the N- and/or C-terminus in SEQ ID NOs: 3, 4, 5, 6, 7 and 8.

9. A medicament comprising one or more peptides or recombinant proteins according to any one of claims 1 to 4, or the polynucleotide according to claim 5.

10. Use of a peptide or a recombinant protein according to any one of claims 1 to 4, or use of the polynucleotide according to claim 5 for the manufacture of a medicament for preventive and/or therapeutic treatment of Alzheimer's disease.

11. An antibody or antibody fragment binding to an antigen specific for a peptide sequence selected from the group consisting of SEQ ID NOs 3, 4, 5, 6, 7, and 8, or a multimer thereof; or a variant thereof wherein 1 or 2 amino acid residues is/are deleted from the N- and/or C-terminus in SEQ ID NOs: 3, 4, 5, 6, 7 and 8, and wherein at least four amino acids in said peptide sequence are part of a reactive portion with said antibody.

12. Use of an antibody or antibody fragment specific for a peptide sequence selected from the group consisting of SEQ ID NOs 3, 4, 5, 6, 7, and 8, or a multimer thereof, or a variant thereof wherein 1 or 2 amino acid residues is/are deleted from the N- and/or C-terminus in SEQ ID NOs: 3, 4, 5, 6, 7 and 8, and wherein at least four amino acids in said peptide sequence are part of a reactive portion with said antibody; wherein said antibody is used in a binding assay for the detection of the β-Amyloid protein or its peptidic fragment β-Amyloid 29-42 (Aβ 29-42) GAIIGLMVGGVVIA (SEQ ID NO: 1).

13. Use of an antibody or antibody fragment specific for a peptide sequence selected from the group consisting of SEQ ID NOs 3, 4, 5, 6, 7, and 8, or a multimer thereof, and wherein at least four amino acids in said peptide sequence are part of a reactive portion with said antibody; wherein said antibody is used in a detection method of the β-Amyloid protein or its peptidic fragment β-Amyloid 29-42 (Aβ 29-42) GAIIGLMVGGVVIA (SEQ ID NO: 1); wherein said method comprises:
- a step of contacting a biological sample containing the β-Amyloid protein or its peptidic fragment β-Amyloid 29-42 (Aβ 29-42) with a peptide or recombinant protein comprising one or a combination of amino acid sequences selected from the group consisting of SEQ ID NOs 3, 4, 5, 6, 7, and 8, or a multimer thereof; or a variant thereof wherein 1 or 2 amino acid residues is/are deleted from the N- and/or C-terminus in SEQ ID NOs: 3, 4, 5, 6, 7 and 8, and
- a step of detecting the binding of said peptide or of said recombinant protein to the β-Amyloid protein or to its β-Amyloid peptidic fragment 29-42 (Aβ 29-42).

14. The product, use or method according to any one of claims 1 to 13, wherein two or more residues are added to the C-terminus after Val in SEQ ID NO: 3, 4, 5, 6, 7 and 8, wherein these residues are selected from the group consisting of Leu-(Gly)ₙ, Ile-(Gly)ₙ, Val-(Gly)ₙ, Nva-(Gly)ₙ, or Nle-(Gly)ₙ, wherein Nva is norvaline, Nle is norleucine, and n=1-10, and/or wherein one or more residues is/are added to the N-terminus before Glu in SEQ ID NO: 3, 4, 5, 6, 7 and 8, wherein these residues are selected from the group consisting of Gly, Lys-(Gly)ₙ, Tyr-(Gly)ₙ, or Gly-(Gly)ₙ, wherein n=1-10.

15. The product, use or method according to any one of claims 1 to 13, wherein any one of the following amino acids in form of a 9-mer addition is added to the C-terminus after Val in SEQ ID NO: 3, 4, 5, 6, 7 and 8 Leu, Ile, Val, Nva, Nle, Met, Ala, and Gly.

16. The product, use or method according to any one of claims 1 to 13, wherein the peptides according to SEQ ID NO: 3, 4, 5, 6, 7 and 8 are derivatized by the following derivatizations selected from the group:
i) Lysinyl and amino terminal residues are derivatized with succinic or other carboxylic acid anhydrides, and/or
ii) α-amino-containing residues are derivatized with a substance selected from the group consisting of imidoesters such as methyl picolinimidate; pyridoxal phosphate; pyridoxal; chloroborohydride; trinitrobenzenesulfonic acid; O-methylisourea; 2,4 pentanedione; and transaminase-catalyzed reaction with glyoxylate;
iii) selectively modifying the carboxyl side aspartyl or glutamyl residues with a substance selected from the group consisting of carbodiimides (R-N=C=N-R') such as 1-cyclohexyl-3-(2-morpholinyl-(4-ethyl) carbodiimide or 1-ethyl-3-(4-azonia-4,4-dimethylpentyl) carbodiimide;
iv) aspartyl and glutamyl residues are converted to asparaginyl or glutaminyl residues by reaction with ammonia;
v) hydroxylation of proline and lysine residues;
vi) phosphorylation of hydroxyl groups of seryl or threonyl residues,
vii) methylation of the amino group of lysine,
viii) acetylation of the N-terminal amine,
ix) amidation of the C-terminal carboxyl groups.

17. The product, use or method according to any one of claims 1 to 13, wherein the peptides according to SEQ ID NO: 3, 4, 5, 6, 7 and 8 are replaced by its respective retro-inverso sequence, wherein the direction of the peptide chain has been inverted and wherein all the amino acids belong to the D-series.

## Patentansprüche

1. Ein isoliertes Peptid, das von dem Peptidfragment 270-287 des Wildtyp-Apolipoproteins E 3 (ApoE WT) mit der Sequenz EDMQRQWAGLVEKVQAAV (SEQ ID NR: 2) abgeleitet ist, wobei das Peptid eine Länge von 18 bis 50 Aminosäureresten besitzt und eine verbesserte Wechselwirkung mit dem β-Amyloid Peptidfragment 29-42 (Aβ 29-42) GAIIGLMVGGVVIA (SEQ ID NR: 1) aufweist, wobei das Peptid eine Aminosäuresequenz oder eine Kombination davon enthält ausgewählt aus der Gruppe bestehend aus den Sequenzen:
-EDMQRQLAGVVEKVQAAV (SEQ ID NR: 3)
-EDMQRQLAGLVEKWQAAV (SEQ ID NR: 4)
-EDMQRQLAGMWEKVQAAV (SEQ ID NR: 5)
-EDVQRQLAGLVEKVQAAV (SEQ ID NR: 6)
-EDMQRQMAGLMEKMQAAV (SEQ ID NR: 7) and
-EDMQRQVAGMWEKVQAAV (SEQ ID NR: 8)
-oder ein Multimer davon, oder eine Variante davon, wobei 1 oder 2 Aminosäurereste des N- und/oder C-Terminus in den Sequenzen SEQ ID NR: 3, 4, 5, 6, 7 und 8 entfernt sind.

2. Das Peptid gemäß Anspruch 1, das wenn es mit dem β-Amyloid Peptidfragment 29-42 (Aβ 29-42) GAIIGLMVGGVVIA (SEQ ID NR: 1) komplexiert ist, die fusionsfördernde Akivität von Aβ 29-42 verringert.

3. Das Peptid gemäß Anspruch 1 oder 2, wobei das Peptid eine Aminosäuresequenz enthält ausgewählt aus der Gruppe bestehend aus:
EDMQRQLAGVVEKVQAAV (SEQ ID NR: 3) und
EDMQRQMAGLMEKMQAAV (SEQ ID NR: 7)
oder ein Multimer davon.

4. Ein rekombinantes Protein, Polypeptide oder Oligopeptide enthaltend ein Peptid oder eine Kombination der Peptide gemäß irgend einem der Ansprüche 1 bis 3.

5. Ein Polynukleotid, das irgend eines der Peptide der Ansprüche 1 bis 3 kodiert oder das rekombinante Protein, Polypeptid oder Oligopeptid von Anspruch 4 kodiert.

6. Ein Verfahren zum Nachweis des β-Amyloid-Proteins oder dessen Peptidfragment β-Amyloid 29-42 (Aβ 29-42) GAIIGLMVGGWIA (SEQ ID NR: 1), wobei das Verfahren umfasst:
- einen Schritt des In-Kontakt-Bringens einer biologischen Probe enthaltend das β-Amyloid-Protein oder dessen Peptidfragment β-Amyloid 29-42 (Aβ 29-42) mit einem Peptid oder einem rekombinanten Protein enthaltend eine Aminosäuresequenz oder eine Kombination davon ausgewählt aus der Gruppe bestehend aus SEQ ID NR: 3, 4, 5, 6, 7, und 8, oder ein Multimer davon; oder eine Variante davon, wobei 1 oder 2 Aminosäurereste des N- und/oder C-Terminus in SEQ ID NR: 3, 4, 5, 6, 7 und 8 entfernt sind, und
- einen Schritt des Nachweises der Bindung des Peptides oder des rekombinanten Proteins an das β-Amyloid-Protein oder an dessen β-Amyloid Peptidfragment 29-42 (Aβ 29-42).

7. Ein Kit zur Verwendung bei dem Nachweis des β-Amyloid-Proteins oder dessen Peptidfragment β-Amyloid 29-42 (Aβ 29-42) GAIIGLMVGGVVIA (SEQ ID NR: 1), wobei das Kit ein Peptid oder ein rekombinantes Protein aufweist enthaltend eine Aminosäuresequenz oder eine Kombination davon ausgewählt aus der Gruppe bestehend aus SEQ ID NR: 3, 4, 5, 6, 7, und 8, oder ein Multimer davon; oder eine Variante davon, wobei 1 oder 2 Aminosäurereste des N- und/oder C-Terminus in SEQ ID NR: 3, 4, 5, 6, 7 und 8 entfernt sind, wobei das Kit weiterhin einen Antikörper enthält zum Nachweis der Bindung des Peptides oder eines rekombinanten Proteins an das β-Amyloid-Protein oder dessen β-Amyloid Peptidfragment 29-42 (Aβ 29-42).

8. Ein Bindungsassay zum Nachweis des β-Amyloid-Proteins oder dessen Peptidfragment β-Amyloid 29-42 (Aβ 29-42) GAIIGLMVGGWIA (SEQ ID NR: 1), wobei der Assay die Verwendung eines Peptides oder eines rekombinanten Proteins beinhaltet, das eine Aminosäuresequenz oder eine Kombination davon enthält ausgewählt aus der Gruppe bestehend aus SEQ ID NR: 3, 4, 5, 6, 7, und 8, oder ein Multimer davon; oder eine Variante davon, wobei 1 oder 2 Aminosäurereste des N- und/oder C-Terminus in SEQ ID NR: 3, 4, 5, 6, 7 und 8 entfernt sind.

9. Ein Arzneimittel enthaltend ein oder mehrere Peptide oder rekombinante Proteine gemäß irgend einem der Ansprüche 1 bis 4, oder das Polynukleotid gemäß Anspruch 5.

10. Verwendung eines Peptides oder eines rekombinanten Proteins gemäß irgend einem der Ansprüche 1 bis 4, oder Verwendung des Polynukleotides gemäß Anspruch 5 zur Herstellung eines Arzneimittels zur vorbeugenden und/oder therapeutischen Behandlung der Alzheimer'-schen Krankheit.

11. Ein Antikörper oder ein Antikörperfragment, das an ein Antigen bindet, das spezifisch für eine Peptidsequenz ist, ausgewählt aus der Gruppe bestehend aus SEQ ID NR: 3, 4, 5, 6, 7, und 8, oder ein Multimer davon; oder eine Variante davon, wobei 1 oder 2 Aminosäurereste des N- und/oder C-Terminus in SEQ ID NR: 3, 4, 5, 6, 7 und 8 entfernt sind, und wobei zumindest vier Aminosäuren in der Peptidsequenz Teil eines reaktiven Bereiches sind, an den der Antikörper bindet.

12. Verwendung eines Antikörper oder eines Antikörperfragmentes, das spezifisch für eine Peptidsequenz ist, ausgewählt aus der Gruppe bestehend aus SEQ ID NR: 3, 4, 5, 6, 7, und 8, oder ein Multimer davon; oder eine Variante davon, wobei 1 oder 2 Aminosäurereste des N- und/oder C-Terminus in SEQ ID NR: 3, 4, 5, 6, 7 und 8 entfernt sind, und wobei zumindest vier Aminosäuren in der Peptidsequenz Teil eines reaktiven Bereiches sind, an den der Antikörper bindet; wobei der Antikörper in einem Bindungsassay zum Nachweis des β-Amyloid-Proteins oder dessen Peptidfragment β-Amyloid 29-42 (Aβ 29-42) GAIIGLMVGGVVIA (SEQ ID NR: 1) verwendet wird.

13. Verwendung eines Antikörper oder eines Antikörperfragmentes, das spezifisch für eine Peptidsequenz ist, ausgewählt aus der Gruppe bestehend aus SEQ ID NR: 3, 4, 5, 6, 7, und 8, oder ein Multimer davon, und wobei zumindest vier Aminosäuren in der Peptidsequenz Teil eines reaktiven Bereiches sind, an den der Antikörper bindet; wobei der Antikörper in einem Bindungsassay zum Nachweis des β-Amyloid-Proteins oder dessen Peptidfragment β-Amyloid 29-42 (Aβ 29-42) GAIIGLMVGGVVIA (SEQ ID NR: 1) verwendet wird; wobei das Verfahren umfasst:
- einen Schritt des In-Kontakt-Bringens einer biologischen Probe enthaltend das β-Amyloid-Protein oder dessen Peptidfragment β-Amyloid 29-42 (Aβ 29-42) mit einem Peptid oder einem rekombinanten Protein enthaltend eine Aminosäuresequenz oder eine Kombination davon ausgewählt aus der Gruppe bestehend aus SEQ ID NR: 3, 4, 5, 6, 7, und 8, oder ein Multimer davon; oder eine Variante davon, wobei 1 oder 2 Aminosäurereste des N- und/oder C-Terminus in SEQ ID NR: 3, 4, 5, 6, 7 und 8 entfernt sind, und
- einen Schritt des Nachweises der Bindung des Peptides oder des rekombinanten Proteins an das β-Amyloid-Protein oder an dessen β-Amyloid Peptidfragment 29-42 (Aβ 29-42).

14. Das Produkt, die Verwendung oder das Verfahren gemäß irgend einem der Ansprüche 1 bis 13, wobei ein oder mehr Reste an den C-Terminus nach Val in den Sequenzen SEQ ID NR: 3, 4, 5, 6, 7 und 8 zugefügt sind, wobei diese Reste ausgewählt sind aus der Gruppe bestehend aus Leu-(Gly)ₙ, Ile-(Gly)ₙ, Val-(Gly)ₙ, Nva-(Gly)ₙ, oder Nle-(Gly)ₙ, wobei Nva Norvalin ist, und Nle Norleucin ist, und n=1-10, und/oder wobei ein oder mehrere Reste an den N-Terminus vor Glu in den Sequenzen SEQ ID NR: 3, 4, 5, 6, 7 und 8 zugefügt sind, wobei diese Reste ausgewählt sind aus der Gruppe bestehend aus Gly, Lys-(Gly)ₙ, Tyr-(Gly)ₙ, oder Gly-(Gly)ₙ, wobei n=1-10.

15. Das Produkt, die Verwendung oder das Verfahren gemäß irgend einem der Ansprüche 1 bis 13, wobei irgend eine der folgenden Aminosäuren in Form einer 9-mer-Addition an den C-Terminus nach Val in den Sequenzen SEQ ID NR: 3, 4, 5, 6, 7 und 8 zugefügt ist und Leu, Ile, Val, Nva, Nle, Met, Ala, und Gly darstellt.

16. Das Produkt, die Verwendung oder das Verfahren gemäß irgend einem der Ansprüche 1 bis 13, wobei die Peptide gemäß SEQ ID NO: 3, 4, 5, 6, 7 und 8 durch folgende Derivatisierungen ausgewählt aus der Gruppe modifiziert sind:
i) Lysinyl und aminoterminale Reste werden durch Bernsteinsäure- oder andere Carbonsäureanhydride derivatisiert, und/oder
ii) α-amino-haltige Reste werden mit einer Substanz ausgewählt aus der Gruppe Imidoester wie Methylpicolinimidat; Pyridoxalphosphat; Pyridoxal; Chlorborhydrid; Trinitrobenzolsulfonsäure; O-Methylisoharnstoff; 2,4 Pentandion; and Transaminase-katalysierte Reaktion mit Glyoxylat derivatisiert;
iii) selektives Modifizieren der carboxylständigen Aspartyl- oder Glutamylreste mit einer Substanz ausgewählt aus der Gruppe bestehend aus Carbodiimide (R-N=C=N-R') wie 1-Cyclohexyl-3-(2-morpholinyl-(4-ethyl) Carbodiimid oder 1-Ethyl-3-(4-azonia-4,4-dimethylpentyl) Carbodiimid;
iv) Aspartyl- and Glutamylreste werden durch Reaktion mit Ammoniak in Asparaginyl- oder Glutaminylreste umgewandelt;
v) Hydroxylierung von Prolin- und Lysinresten;
vi) Phosphorylierung der Hydroxylgruppen der Seryl- oder Threonylreste,
vii) Methylierung der Aminogruppe von Lysin,
viii) Acetylierung des N-terminalen Amins,
ix) Amidierung der C-terminalen Carboxylgruppen.

17. Das Produkt, die Verwendung oder das Verfahren gemäß irgend einem der Ansprüche 1 bis 13, wobei die Peptide gemäß SEQ ID NR: 3, 4, 5, 6, 7 und 8 durch ihre entsprechende retro-inverse Sequenz ersetzt werden, wobei die Orientierung der Peptidkette invertiert ist und wobei alle Aminosäuren D-Aminosäuren sind.

## Revendications

1. Peptide isolé dérivé du fragment peptidique de l'apolipoprotéine E3 de type sauvage 270-287 (ApoE WT) de séquence EDMQRQWAGLVEKVQAAV (SEQ ID NO : 2), dans lequel le peptide a une longueur comprise entre 18 et 50 résidus d'acides aminés et présente une interaction améliorée avec le fragment peptidique β-Amyloïde 29-42 (Aβ 29-42) GAIIGLMVGGWIA (SEQ ID NO : 1), ledit peptide comprenant une séquence d'acides aminés ou une association de séquences d'acides aminés sélectionnées dans le groupe constitué de :
- EDMQRQLAGWEKVQAAV (SEQ ID NO : 3)
- EDMQRQLAGLVEKWQAAV (SEQ ID NO : 4)
- EDMQRQLAGMWEKVQAAV (SEQ ID NO : 5)
- EDVQRQLAGLVEKVQAAV (SEQ ID NO : 6)
- EDMQRQMAGLMEKMQAAV (SEQ ID NO : 7) et
- EDMQRQVAGMWEKVQAAV (SEQ ID NO : 8)
- ou un multimère de celui-ci, ou un variant de celui-ci dans lequel 1 ou 2 résidu(s) d'acides aminés est/sont supprimé(s) de l'extrémité N-terminale et/ou C-terminale dans les SEQ ID NO : 3, 4, 5, 6, 7 et 8.

2. Peptide selon la revendication 1, ce peptide, lorsqu'il est complexé au fragment peptidique β-Amyloïde 29-42 (Aβ 29-42) GAIIGLMVGGWIA (SEQ ID NO:1), diminue l'activité fusogène de Aβ 29-42.

3. Peptide selon la revendication 1 ou 2, dans lequel ledit peptide comprend une séquence d'acides aminés sélectionnée dans le groupe constitué de :
EDMQRQLAGVVEKVQAAV (SEQ ID NO : 3) et
EDMQRQMAGLMEKMQAAV (SEQ ID NO : 7)
ou un multimère de celui-ci.

4. Protéine, polypeptide ou oligopeptide recombinant comprenant un peptide ou une association des peptides selon l'une quelconque des revendications 1 à 3.

5. Polynucléotide codant l'un quelconque des peptides selon la revendication 1 à 3 ou codant la protéine, le polypeptide ou l'oligopeptide recombinant selon la revendication 4.

6. Procédé de détection de la protéine β-Amyloïde ou de son fragment peptidique β-Amyloïde 29-42 (Aβ 29-42) GAIIGLMVGGVVIA (SEQ ID NO : 1), dans lequel ledit procédé comprend :
- une étape consistant à mettre en contact un échantillon biologique contenant la protéine β-Amyloïde ou son fragment peptidique β-amyloïde 29-42 (Aβ 29-42) avec un peptide ou une protéine recombinante comprenant une séquence d'acides aminés ou une association de séquences d'acides aminés sélectionnées dans le groupe constitué des SEQ ID NO 3, 4, 5, 6, 7 et 8, ou un multimère de celui-ci/celle-ci ; ou un variant de celui-ci/celle-ci dans lequel 1 ou 2 résidu(s) d'acides aminés est/sont supprimé(s) de l'extrémité N-terminale et/ou C-terminale dans les SEQ ID NO : 3, 4, 5, 6, 7 et 8, et
- une étape consistant à détecter la liaison dudit peptide ou de ladite protéine recombinante avec la protéine β-Amyloïde ou avec son fragment peptidique β-Amyloïde 29-42 (Aβ 29-42).

7. Kit destiné à la détection de la protéine β-Amyloïde ou de son fragment peptidique β-Amyloïde 29-42 (Aβ 29-42) GAIIGLMVGGVVIA (SEQ ID NO : 1) le kit comprenant un peptide ou une protéine recombinante comprenant une séquence d'acides aminés ou une association de séquences d'acides aminés sélectionnées dans le groupe constitué des SEQ ID NO 3, 4, 5, 6, 7 et 8, ou un multimère de celui-ci/celle-ci ; ou un variant de celui-ci/celle-ci dans lequel 1 ou 2 résidu(s) d'acides aminés est/sont supprimé(s) de l'extrémité N-terminale et/ou C-terminale dans les SEQ ID NO : 3, 4, 5, 6, 7 et 8, le kit comprenant en outre un anticorps destiné à détecter la liaison dudit peptide ou de ladite protéine recombinante avec la protéine β-Amyloïde ou avec son fragment peptidique β-Amyloide 29-42 (Aβ 29-42).

8. Essai de liaison pour la détection de la protéine β-Amyloïde ou de son fragment peptidique β-Amyloïde 29-42 (Aβ 29-42) GAIIGLMVGGVVIA (SEQ ID NO : 1), l'essai impliquant l'utilisation d'un peptide ou d'une protéine recombinante comprenant une séquence d'acides aminés ou une association de séquences d'acides aminés sélectionnées dans le groupe constitué des SEQ ID NO 3, 4, 5, 6, 7 et 8, ou un multimère de celui-ci/celle-ci, ou un variant de celui-ci/celle-ci dans lequel 1 ou 2 résidu(s) d'acides aminés est/sont supprimé(s) de l'extrémité N-terminale et/ou C-terminale dans les SEQ ID NO : 3, 4, 5, 6, 7 et 8.

9. Médicament comprenant un ou plusieurs peptides ou protéines recombinantes selon l'une quelconque des revendications 1 à 4, ou le polynucléotide selon la revendication 5.

10. Utilisation d'un peptide ou d'une protéine recombinante selon l'une quelconque des revendications 1 à 4, ou utilisation du polynucléotide selon la revendication 5 pour la fabrication d'un médicament destiné au traitement préventif et/ou thérapeutique de la maladie d'Alzheimer.

11. Anticorps ou fragment d'anticorps lié à un antigène spécifique à une séquence de peptides sélectionnée dans le groupe constitué des SEQ ID NO 3, 4, 5, 6, 7 et 8, ou un multimère de celui-ci ; ou un variant de celui-ci dans lequel 1 ou 2 résidu(s) d'acides aminés est/sont supprimé(s) de l'extrémité N-terminale et/ou C-terminale dans les SEQ ID NO : 3, 4, 5, 6, 7 et 8, et dans lequel au moins quatre acides aminés dans ladite séquence de peptides font partie d'une partie réactive avec ledit anticorps.

12. Utilisation d'un anticorps ou d'un fragment d'anticorps spécifique à une séquence de peptides sélectionnée dans le groupe constitué des SEQ ID NO 3, 4, 5, 6, 7 et 8, ou un multimère de celui-ci ; ou un variant de celui-ci dans lequel 1 ou 2 résidu(s) d'acides aminés est/sont supprimé(s) de l'extrémité N-terminale et/ou C-terminale dans les SEQ ID NO : 3, 4, 5, 6, 7 et 8, et dans laquelle au moins quatre acides aminés dans ladite séquence de peptides font partie d'une partie réactive avec ledit anticorps ; dans laquelle ledit anticorps est utilisé dans un essai de liaison pour la détection de la protéine β-Amyloïde ou de son fragment peptidique β-Amyloïde 29-42 (Aβ 29-42) GAIIGLMVGGWIA (SEQ ID NO:1).

13. Utilisation d'un anticorps ou d'un fragment d'anticorps spécifique à une séquence de peptides sélectionnée dans le groupe constitué des SEQ ID NO 3, 4, 5, 6, 7 et 8, ou un multimère de celui-ci, et dans laquelle au moins quatre acides aminés dans ladite séquence de peptides font partie d'une partie réactive avec ledit anticorps ; dans laquelle ledit anticorps est utilisé dans un procédé de détection de la protéine β-Amyloïde ou de son fragment peptidique β-Amyioïde 29-42 (Aβ 29-42) GAIIGLMVGGWIA (SEQ ID NO : 1); dans laquelle ledit procédé comprend :
- une étape consistant à mettre en contact un échantillon biologique contenant la protéine β-Amyloïde ou son fragment peptidique β-Amyloïde 29-42 (Aβ 29-42) avec un peptide ou une protéine recombinante comprenant une séquence d'acides aminés ou une association de séquences d'acides aminés sélectionnées dans le groupe constitué des SEQ ID NO 3, 4, 5, 6, 7 et 8, ou un multimère de celui-ci/celle-ci ; ou un variant de celui-ci/celle-ci dans lequel 1 ou 2 résidu(s) d'acides aminés est/sont supprimé(s) de l'extrémité N-terminale et/ou C-terminale dans les SEQ ID NO : 3, 4, 5, 6, 7 et 8, et
- une étape consistant à détecter la liaison dudit peptide ou de ladite protéine recombinante avec la protéine β-Amyloïde ou avec son fragment peptidique β-Amyloïde 29-42 (Aβ 29-42).

14. Produit, utilisation ou procédé selon l'une quelconque des revendications 1 à 13, dans lequel au moins deux résidus sont ajoutés à l'extrémité C-terminale après Val dans les SEQ ID NO : 3, 4, 5, 6, 7 et 8, dans lequel ces résidus sont sélectionnés dans le groupe constitué de Leu-(Gly)ₙ, de Ile-(Gly)ₙ, de Val-(Gly)ₙ, de Nva-(Gly)ₙ ou de Nle-(Gly)ₙ, dans lequel Nva est de la norvaline, Nle est de la norleucine et n=1-10, et/ou dans lequel un ou plusieurs résidus est/sont ajouté(s) à l'extrémité N-terminale avant Glu dans les SEQ ID NO : 3, 4, 5, 6, 7 et 8, dans lequel ces résidus sont sélectionnés dans le groupe constitué de Gly, de Lys-(Gly)ₙ, de Tyr-(Gly)ₙ ou de Gly-(Gly)ₙ, dans lequel n=1-10.

15. Produit, utilisation ou procédé selon l'une quelconque des revendications 1 à 13, dans lequel l'un quelconque des acides aminés suivants, en forme d'ajout de 9-mer, est ajouté à l'extrémité C-terminale après Val dans les SEQ ID NO : 3, 4, 5, 6, 7 et 8 Leu, Ile, Val, Nva, Nle, Met, Ala et Gly.

16. Produit, utilisation ou procédé selon l'une quelconque des revendications 1 à 13, dans lequel les peptides selon les SEQ ID NO : 3, 4, 5, 6, 7 et 8 sont dérivés par les dérivations suivantes sélectionnées dans le groupe suivant :
i) les résidus lysinyle et amino-terminaux sont dérivés avec des anhydrides d'acides succiniques ou autres acides carboxyliques, et/ou
ii) les résidus contenant des acides α-aminés sont dérivés avec une substance sélectionnée dans le groupe constitué d'imidoesters tels que le picolinimidate de méthyle ; le phosphate de pyridoxal ; le pyridoxal ; le chloroborohydrure ; l'acide trinitrobenzènesulfonique ; l'O-méthylisourée ; le 2,4-pentanedione ; et la réaction catalysée par la transaminase avec du glyoxylate ;
iii) la modification sélective des résidus aspartyle ou glutamyle latéraux carboxyle avec une substance sélectionnée dans le groupe constitué de carbodiimides (R-N=C=N-R') tels que le carbodiimide 1-cyclohexyle-3-(2-morpholinyle-(4-éthyle) ou le carbodiimide 1-éthyle-3-(4-azonia-4,4-diméthylpentyl) ;
iv) les résidus aspartyle et glutamyle sont convertis en résidus asparaginyle ou glutaminyle par réaction avec l'ammoniac ;
v) l'hydroxylation de résidus proline et lysine ;
vi) la phosphorylation de groupes hydroxyle de résidus séryle ou thréonyle,
vii) la méthylation du groupe aminé de lysine,
viii) l'acétylation de l'amine N-terminale,
ix) l'amidation des groupes carboxyle C-terminaux.

17. Produit, utilisation ou procédé selon l'une quelconque des revendications 1 à 13, dans lequel les peptides selon les SEQ ID NO : 3, 4, 5, 6, 7 et 8 sont remplacés par sa séquence rétro-inverso respective, dans lequel le sens de la chaîne peptidique a été inversé et dans lequel tous les acides aminés appartiennent à la série D.
